# EUROPEAN PATENT APPLICATION

(11) **EP 2 487 177 A1**
(43) Date of publication of application: **15.08.2012**
(21) Application number: 10822057.5
(22) Date of filing: 06.10.2010
(51) Int. Cl.: C07D 491/048, A61K 31/4162, A61P 1/04, A61P 9/00, A61P 9/12, A61P 25/00, A61P 25/14, A61P 25/18, A61P 25/20, A61P 25/22, A61P 25/24, A61P 25/28, A61P 25/30, A61P 25/32, A61P 43/00

(54) **PYRAZOLOXAZOLE COMPOUND**

(30) Priority: 08.10.2009 JP 2009234111; 08.10.2009 US 249741 P
(71) Applicant: Eisai R&D Management Co., Ltd., Tokyo 112-8088 (JP)
(72) Inventor: SHIN Kogyoku, Hatfield Hertfordshire AL10 9SN (GB); TERAUCHI Taro, Tsukuba-shi Ibaraki 300-2635 (JP); TAKAHASHI Yoshinori, Tsukuba-shi Ibaraki 300-2635 (JP); HASHIZUME Minako, Tsukuba-shi Ibaraki 300-2635 (JP); TAKEDA Kunitoshi, Tsukuba-shi Ibaraki 300-2635 (JP); SHIKATA Kodo, Hatfield Hertfordshire AL10 9SN (GB); INOMATA Akira, Tsukuba-shi Ibaraki 300-2635 (JP)
(74) Representative: HOFFMANN EITLE
(86) International application number: PCT/JP2010/067564
(87) International publication number: WO 2011/043387

(57) **Abstract**

A compound represented by the formula (I) or pharmacologically acceptable salt thereof exhibits an excellent CRF receptor antagonism. 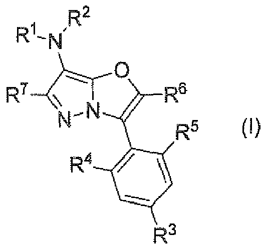 wherein R¹ and R² are the same or different and are a hydrogen atom, a C1-6 alkyl group, a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, etc; R³, R⁴ and R⁵ are the same or different and are a hydrogen atom, a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom; R⁶ is a hydrogen atom or a C1-6 alkyl group; and R⁷ is a C1-6 alkyl group, a C1-6 alkoxy group or a C1-6 alkylthio group.

## Description

### Technical Field

The present invention relates to novel compounds having corticotropin-releasing factor (hereinafter, referred to as "CRF") receptor antagonistic activity, and pharmacologically acceptable salts thereof and to medical use of the same.

### Background Art

CRF is a neuropeptide that consists of 41 amino acids and is produced and secreted in the hypothalamus and promotes release of adrenocorticotropic hormone (ACTH) under stress, and it also functions in the brain as a neurotransmitter or a neuromodulator, integrating electrophysiology, autonomic nerves, behavior, and the like, in response to stress.

There are two subtypes in CRF receptors, CRF1 receptor and CRF2 receptor, and CRF1 receptor has been reported to be widely distributed in the cerebral cortex, cerebellum, olfactory bulb, pituitary gland, amygdaloid nucleus, and the like.
Furthermore, many low molecular compounds having CRF receptor antagonism have been noted as potential therapeutic agents for a variety of diseases including depression, anxiety, stress-related disorders, and the like (see Non-patent Document 1).

Disclosed compounds having CRF receptor antagonism include compounds having a 2,6-dimethoxy-4-methoxymethylphenyl group (see Patent Document 1), but compounds having a pyrazolo[5,1-b]oxazole skeleton according to the invention of the present application have been neither disclosed nor suggested.

As a compound having a pyrazolo[5,1-b]thiazole skeleton, the compound shown below has been disclosed but its use is for colorimetry (see Example 16 of Patent Document 2).

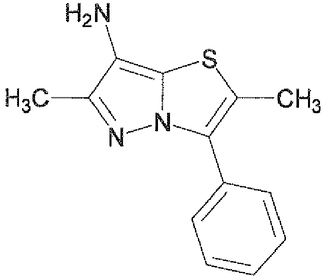

Patent Document 3 (international filing date: October 22, 2009) discloses the following compounds which have a pyrazolo[5,1-b]thiazole skeleton and have CRF receptor antagonism. 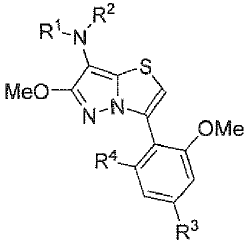 wherein R¹ is the formula -A¹¹-A¹², R² is tetrahydrofurylmethyl, tetrahydropyranylmethyl or tetrahydropyranyl, A¹¹ is a single bond, methylene or 1,2-ethylene, A¹² is C1-6 alkyl, C3-6 cycloalkyl or C3-6 cycloalkyl having methyl, R³ is methoxy, cyano, cyclobutyloxymethyl, methoxymethyl or ethoxymethyl, and R⁴ is methoxy or chlorine.

Patent Document 4 (international filing date: October 22, 2009) discloses the following compounds which have a pyrazolo[5,1-b]oxazole skeleton and have CRF receptor antagonism. 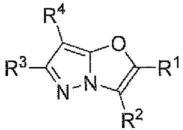 wherein R¹ and R³ are the same or different and are a hydrogen atom, C 1-6 alkyl or C1-6 haloalkyl; R² is phenyl, a 5- or 6-membered heteroaryl or a bicyclic heteroaryl group, each of which may optionally be substituted; and R⁴ is -OR⁷, -(CH2)ₘNR⁸R⁹ or -COR¹⁰, etc.
Both of Patent Documents 3 and 4 were published after the filing date of the earliest priority applications (Japanese patent application No. 2009-234111 and U.S. provisional application No. 61/249741; both were filed on October 8, 2009) of the present application.

### Citation List

### Patent Literature

[Patent document 1] U.S. Patent Application Publication No. 2004/0224974
[Patent document 2] U.S. Patent No. 5,234,818
[Patent document 3] WO 2009/128383
[patent document 4] WO 2010/015628

### Non Patent Literature

[Non-patent document 1] Drugs of the Future, 24:1089-1098 (1999)

### Summary of Invention

### Technical Problem

No 3-phenylpyrazolo[5,1-b]oxazole compounds having superior CRF receptor antagonism are known. Furthermore, although compounds having CRF receptor antagonism have been reported, they have not necessarily been sufficient in terms of having superior CRF receptor antagonism, and in terms of having sufficient pharmacological activity, safety and pharmacokinetic properties as medicines.

### Solution to Problem

In view of the above-mentioned current circumstances, the present inventors have intensively studied and, as a result, have discovered novel compounds that are excellent CRF receptor antagonists with sufficient pharmacological activity, safety, and pharmacokinetics, and are useful as prophylactic agents or therapeutic agents for diseases such as depression, anxiety, and irritable bowel syndrome.

Specifically, the present invention relates to the following <1> to <22>.
<1> A compound represented by the formula (I) or pharmacologically acceptable salt thereof: 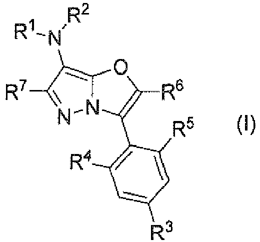 wherein R¹ and R² are the same or different and are (a) a hydrogen atom, (b) a C1-6 alkyl group, (c) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, (d) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (e) R¹ and R², together with a nitrogen atom to which they are attached, form a pyrrolidinyl group, a piperidinyl group or a morpholinyl group;
   R³, R⁴ and R⁵ are the same or different and are a hydrogen atom, a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
   R⁶ is a hydrogen atom or a C1-6 alkyl group; and
   R⁷ is a C1-6 alkyl group, a C1-6 alkoxy group or a C1-6 alkylthio group.
<2> The compound or pharmacologically acceptable salt thereof according to <1>,
   wherein R¹ is a hydrogen atom, a C1-6 alkyl group, a C3-6 cycloalkyl group or a C3-6 cycloalkyl-C1-6 alkyl group;
   R² is (a) a hydrogen atom, (b) a C1-6 alkyl group, (c) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (d) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group;
   R³ is a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
   R⁴ is a hydrogen atom, a C1-6 alkyl group or a C 1-6 alkoxy group; and
   R⁵ is a halogen atom, a C 1-6 alkyl group or a C1-6 alkoxy group.
<3> The compound or pharmacologically acceptable salt thereof according to <2>,
   wherein R¹ is a hydrogen atom, a C1-6 alkyl group, a cyclopropyl group or a cyclopropylmethyl group; and
   R² is a hydrogen atom, a C1-6 alkyl group, a cyclopropylmethyl group, a tetrahydropyranyl group, a tetrahydropyranylmethyl group, a dioxanylmethyl group, or a tetrahydrofurylmethyl group.
<4> The compound or pharmacologically acceptable salt thereof according to <2>,
   wherein R³ is a halogen atom, methoxy or a C1-6 alkoxymethyl group;
   R⁴ is a hydrogen atom or a methoxy group; and
   R⁵ is a halogen atom or a methoxy group.
<5> The compound or pharmacologically acceptable salt thereof according to <2>,
   wherein R⁶ is a hydrogen atom or a methyl group; and
   R⁷ is a methyl group or an ethyl group.
<6> The compound or pharmacologically acceptable salt thereof according to <1>,
   wherein R¹ is a hydrogen atom, a C1-6 alkyl group, a cyclopropyl group or a cyclopropylmethyl group;
   R² is a hydrogen atom, a C1-6 alkyl group, a cyclopropylmethyl group, a tetrahydropyranyl group, a tetrahydropyranylmethyl group, a dioxanylmethyl group or a tetrahydrofurylmethyl group;
   R³ is a halogen atom, a methoxy group or a C1-6 alkoxymethyl group;
   R⁴ is a hydrogen atom or a methoxy group;
   R⁵ is a halogen atom or a methoxy group;
   R⁶ is a hydrogen atom or a methyl group; and
   R⁷ is a methyl group or an ethyl group.
<7> A pharmaceutical composition comprising a compound or pharmacologically acceptable salt thereof according to <1> as an active ingredient.
<8> The pharmaceutical composition according to <7>, which is a CRF1 receptor antagonist.
<9> A therapeutic or a prophylactic agent for depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia, comprising a compound or pharmacologically acceptable salt thereof according to <1> as an active ingredient.
<10> A therapeutic or prophylactic agent for depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction or dementia, comprising a compound or pharmacologically acceptable salt thereof according to <1> as an active ingredient.
<11> A therapeutic or a prophylactic agent for depression, depressive symptoms, anxiety or irritable bowel syndromes, comprising a compound or pharmacologically acceptable salt thereof according to <1> as an active ingredient.
<12> A compound represented by the formula (Ia) or pharmacologically acceptable salt thereof: 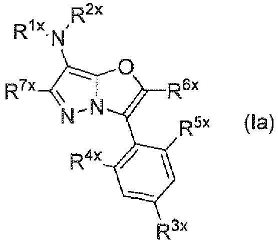 wherein R^{1x} and R^{2x} are the same or different and are (a) a C1-6 alkyl group, (b) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, (c) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (d) R^{1x} and R^{2x}, together with a nitrogen atom to which they are attached, form a pyrrolidinyl group, a piperidinyl group or a morpholinyl group;
   R^{3x}, R^{4x} and R^{5x} are the same or different and are a hydrogen atom, a C1-6 alkyl group, C3-6 cycloalkyl, a C1-6 alkoxy group, a C1-6 alkoxy-C1-6 alkyl group, a C3-6 cycloalkoxy-C1-6 alkyl group or a halogen atom;
   R^{6x} is a hydrogen atom or a C1-6 alkyl group; and
   R^{7x} is a C1-6 alkyl group, a C1-6 alkoxy group or a C1-6 alkylthio group,
   with the proviso that when R^{7x} is a C1-6 alkyl group, at least one of R^{1x} and R^{2x} is (a) a cyclic group selected from a tetrahydropyranyl group, a dihydropyranyl group, a tetrahydrofuryl group and a dioxanyl group or (b) a C1-6 alkyl group substituted wiht a cyclic group selected from a tetrahydropyranyl group, a dihydropyranyl group, a tetrahydrofuryl group and a dioxanyl group.
<13> The compound or pharmacologically acceptable salt thereof according to <12>,
   wherein R^{1x} is a C1-6 alkyl group, a C3-6 cycloalkyl group or a C3-6 cycloalkyl-C1-6 alkyl group;
   R^{2x} is (a) a cyclic group selected from a tetrahydropyranyl group, a dihydropyranyl group, a tetrahydrofuryl group and a dioxanyl group or (b) a C1-6 alkyl group substituted with a cyclic group selected from a tetrahydropyranyl group, a dihydropyranyl group, a tetrahydrofuryl group and a dioxanyl group;
   R^{3x} is a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
   R^{4x} is a hydrogen atom, a C1-6 alkyl group or a C1-6 alkoxy group; and
   R^{5x} is a halogen atom, a C1-6 alkyl group or a C1-6 alkoxy group.
<14> The compound or pharmacologically acceptable salt thereof according to <13>,
   wherein R^{1x} is a C1-6 alkyl group, a cyclopropyl group or a cyclopropylmethyl group; and
   R^{2x} is a tetrahydropyranyl group, a tetrahydropyranylmethyl group, a dioxanylmethyl group or a tetrahydrofurylmethyl group.
<15> The compound or pharmacologically acceptable salt thereof according to <13>,
   wherein R^{3x} is a halogen atom, a methoxy group or a C1-6 alkoxymethyl group;
   R^{4x} is a hydrogen atom or a methoxy group; and
   R^{5x} is a halogen atom or a methoxy group.
<16> The compound or pharmacologically acceptable salt thereof according to <15>, wherein R^{3x} is a C1-6 alkoxymethyl group.
<17> The compound or pharmacologically acceptable salt thereof according to <13>,
   wherein R^{6x} is a hydrogen atom or a methyl group; and
   R^{7x} is a methyl group or an ethyl group.
<18> The compound or pharmacologically acceptable salt thereof according to <13>,
   wherein R^{1x} is a C1-6 alkyl group, a cyclopropyl group or a cyclopropylmethyl group;
   R^{2x} is a tetrahydropyranyl group, a tetrahydropyranylmethyl group, a dioxanylmethyl group or a tetrahydrofurylmethyl group;
   R^{3x} is a C1-6 alkoxymethyl group,
   R^{4x} is a hydrogen atom or a methoxy group;
   R^{5x} is a halogen atom or a methoxy group;
   R^{6x} is a hydrogen atom or a methyl group; and
   R^{7x} is a methyl group or an ethyl group.
<19> A method for treating or preventing depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia, comprising administering a compound or pharmacologically acceptable salt thereof according to <1> to a patient.
<20> Use of a compound or pharmacologically acceptable salt thereof according to <1> for the manufacture of a therapeutic or prophylactic agent for depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia.
<21> A compound or pharmacologically acceptable salt thereof according to <1> for treating or preventing depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia.
<22> Use of a compound or t pharmacologically acceptable salt thereof according to <1> for treating or preventing depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia.

### Advantageous Effects of Invention

CRF receptor antagonists have been reported to be effective for a variety of diseases as mentioned below.

### (1) Depression, Depressive Symptoms, Anxiety

CRF1 receptor antagonist R121919 is effective for ameliorating depression, depressive symptoms, anxiety, and the like (Journal of Psychiatric Research, 34:171-181 (2000)).
CRF1 receptor antagonist R121919 exhibits an anti-anxiety action in rats (European Journal of Neuroscience, 13:373-380 (2001)).
CRF1 receptor antagonist CP-154526 exhibits anti-depressant and anti-anxiety actions in rats. (European Journal of Pharmacology, 492:195-201 (2004)).

### (2) Irritable Bowel Syndrome (IBS)

CRF1 receptor antagonist α-helical CRF (9-41) inhibits colon intestinal hyperkinesis in IBS patients and reduces abdominal pain and anxiety (Gut 2004; 53:958-964).

### (3) Sleep Disorder, Insomnia

CRF1 receptor antagonist R121919 inhibits stress-related sleep disorder particularly in high-anxiety rats (Journal of Psychiatric Research, 36:197-208 (2002)).

### (4) Alcohol Dependence, Alcohol Withdrawal Symptoms, Drug Dependence, Drug Withdrawal Symptoms

CRF1 receptor antagonist CP-154526 inhibits recurrence of stress-elicited alcohol-seeking behavior in rats (psychopharmacology, 150:317-324 (2000)).
CRF1 receptor antagonist α-helical CRF (9-41) inhibits anxiety behavior in ethanol withdrawal rats (Brain Research, 605:25-32 (1993)).
CRF1 receptor antagonist CP-154526 inhibits recurrence of stress-elicited drug (heroin, cocaine)-seeking behavior in rats (psychopharmacology, 137:184-190 (1998)).
Pretreatment of CRF1 receptor antagonist CP-154526 inhibits naltrexone-induced morphine withdrawal symptoms (Journal of Neurochemistry, 74: 199-208 (2000)).

### (5) Stress-Related Gastrointestinal Dysfunction

CRF1 receptor antagonist NBI-27914 inhibits water avoidance stress-related rat catharsis (Brain Research, 893:29-35 (2001)).

### (6) Anorexia Nervosa, Eating Disorder

CRF1 receptor antagonists α-helical CRF (9-41) and CRA1000 inhibit stress-related reduction in food intake (Brain Research, 823:221-225 (1999)).

### (7) Postoperative Ileus

CRF1 receptor antagonist CP-154526 recovers gastric emptying retardation after surgery (Gastroenterology, 125:654-659 (2003)).

### (8) Dementia, Senile Dementia of Alzheimer type, Multi-infarct Dementia, Senile Dementia

CRF1 receptor antagonist CP-154526 inhibits learning disability following acute stress (Behavioural Brain Research, 138:207-213 (2003)).

CRF1 receptor antagonist α-helical CRF (9-41) suppresses stress-related increase in intracerebral amyloid-β (Proceedings of the National Academy of Sciences of the United States of America, 104: 10673-10678 (2007)).

CRF1 receptor antagonist NBI27914 inhibits increased levels of Aβ and Aβ plaque deposition induced by stress in Tg2576 transgenic mice (Journal of Neurochemistry, 108: 165-175 (2009)).

CRF1 receptor antagonist antalarmin inhibits stress-induced hippocampal tau phosphorylation (Journal of Neuroscience, 27 (24): 6552-6562 (2007)).

### (9) Ischemic Neuropathy, Apoplexy

CRF1 receptor antagonist α-helical CRF (9-41) inhibits ischemic and excitotoxic encephalopathy (Brain Research, 656: 405-408 (1994)).

### (10) Excitotoxic Neuropathy

CRF1 receptor antagonist Asressin inhibits kainic acid-induced excitotoxic neuropathy (Brain Research, 744: 166-170 (1997)).

### (11) Convulsion, Epilepsy

CRF1 receptor antagonist NBI27914 inhibits limbic system seizure (convulsion and epilepsy induced by CRF administration) (Brain Research, 770:89-95 (1997)).

### (12) Hypertension

CRF1 receptor antagonist antalarmin inhibits hypertension induced by intraventricular administration of CRF (Brain Research, 881: 204-207 (2000)).

The compounds or pharmacologically acceptable salts thereof according to the present invention have excellent CRF receptor antagonism, as shown in the activity data in the Pharmacological Test Examples described below. Thus, based on the above-mentioned documents demonstrating a nexus between CRF receptor antagonism and effects of treating or preventing diseases, the compounds or pharmacologically acceptable salts thereof according to the present invention are useful for treatment or prevention of diseases associated with CRF and/or CRF receptors, and are particularly useful as therapeutic agents or prophylactic agents for depression, depressive symptoms, anxiety, irritable bowel syndrome, sleep disorder, insomnia, alcohol dependence, alcohol withdrawal symptoms, drug dependence, drug withdrawal symptoms, stress-related gastrointestinal dysfunction, anorexia nervosa, eating disorder, postoperative ileus, ischemic neuropathy, apoplexy, excitotoxic neuropathy, convulsion, epilepsy, hypertension, schizophrenia, bipolar disorder or dementia, etc.

### Description of Embodiments

Hereinafter, the present invention is described in detail.

In the present specification, the structural formulae for compounds will show a certain isomer for convenience, but the present invention includes all isomers such as geometric isomers, optical isomers, stereoisomers, and tautomers generated by the compound structures, as well as their isomer mixtures, and the compounds may not be limited to the formulae that are shown for convenience and may be any of the isomers or mixtures including isomers in any arbitrary proportions. Thus, for example, the compounds of the present invention may exist as optically active substances or racemic mixtures, but they are not limited to any of them, they may be racemic mixtures or optically active substances, and they may also be mixtures with the optically active substances in any arbitrary ratio.

The present invention may include polymorphic crystals, but similarly include single substances of any crystal forms or a mixture thereof without any restrictions, as well as it may include amorphous forms, and the compounds of the present invention also include both anhydrate and solvate (especially, hydrate). The present invention further encompasses metabolites of compound (I) according to the present invention that are produced by metabolism (oxidation, reduction, hydrolysis, conjugation, and the like) in the living body. The present invention still further encompasses compounds that produce the compound (I) according to the present invention by metabolism (oxidation, reduction, hydrolysis, conjugation, and the like) in the living body (so-called prodrugs).

Hereinafter, the meanings of the terms and symbols used throughout the present specification are described, and the present invention is described in detail.

The term "halogen atom" used in the present specification means a fluorine atom, a chlorine atom, a bromine atom or an iodine atom. Preferable example of the "halogen atom" can include a fluorine atom, and a chlorine atom.

The term "C1-6 alkyl group" used in the present specification means C1-6 straight- or branched-chain alkyl groups, and the specific examples thereof may include a methyl group, an ethyl group, a 1-propyl group (a n-propyl group), a 2-propyl group (an i-propyl group), a 2-methyl-1-propyl group (an i-butyl group), a 2-methyl-2-propyl group (a tert-butyl group), a 1-butyl group (an n-butyl group), a 2-butyl group (an s-butyl group), a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group, a 2,3-dimethyl-2-butyl group, and the like.

The term "C1-6 alkoxy group" used in the present specification means an oxygen atom to which the above-defined "C1-6 alkyl group" is bonded, and specific examples thereof may include a methoxy group, an ethoxy group, a 2-propyloxy group, a 1-pentyloxy group, a 1-hexyloxy group, and the like.

The term "C1-6 alkoxy C1-6 alkyl group" used in the present specification means the above-defined "C1-6 alkyl group" to which the above-defined "C1-6 alkoxy group" is bonded, and specific examples thereof may include a methoxy methyl group, an ethoxymethyl group, a 2-methoxyethyl group, a (2-propyloxy)methyl group, a 6-hexyloxyhexyl group, and the like.

The term "C1-6 alkylthio group" used in the present specification means a sulfur atom to which the above-defined "C1-6 alkyl group" is bonded, and specific examples thereof may include a methylthio group, an ethylthio group, a 2-propylthio group, a 1-pentylthio group, a 1-hexylthio group, and the like.

The term "C3-6 cycloalkyl group" used in the present specification means a monocyclic saturated aliphatic hydrocarbon group having 3 to 6 carbon atoms, and specific examples thereof may include a cyclopropyl group, a cyclobutyl group, a cyclopentyl group, and a cyclohexyl group.

The term "C3-6 cycloalkyl C1-6 alkyl group" used in the present specification means the above-defined "C1-6 alkyl group" to which the above-defined "C3-6 cycloalkyl group" is bonded, and specific examples thereof may include a cyclopropylmethyl group, a cyclobutylmethyl group, a 2-cyclopropylethyl group, and the like.

The term "C3-6 cycloalkoxy group" used in the present specification means an oxygen atom to which the above-defined "C3-6 cycloalkyl group" is bonded, and specific examples thereof may include a cyclopropyloxy group, a cyclobutyloxy group, a cyclopentyloxy group, a cyclohexyloxy group, and the like.

The term "C3-6 cycloalkoxy C1-6 alkyl group" used in the present specification means the above-defined "C1-6 alkyl group" to which the above-defined "C3-6 cycloalkoxy group" is bonded, and specific examples thereof may include a cyclopropyloxymethyl group, a cyclobutyloxymethyl group, 1-(cyclopropyloxy)ethyl group, a cyclohexyloxymethyl group, and the like.

Specific examples of a "tetrahydropyranyl group" used in the present specification may include a tetrahydropyran-4-yl group and a tetrahydropyran-3-yl group, and a preferable example is a tetrahydropyran-4-yl group.

Specific examples of a "tetrahydropyranylmethyl group" used in the present specification may include a (tetrahydropyran-4-yl)methyl group, a (tetrahydropyran-3-yl)methyl group, a (tetrahydropyran-2-yl)methyl group, and a preferable example is a (tetrahydropyran-4-yl)methyl group.

Specific examples of a "tetrahydrofurylmethyl group" used in the present specification may include a (tetrahydrofuran-3-yl)methyl group and a (tetrahydrofuran-2-yl)methyl group, and a preferable example is a (tetrahydrofuran-3-yl)methyl group.

Specific examples of a "dioxanylmethyl group" used in the present specification may include a (1,4-dixan-2-yl)methyl group, a (1,3-dixan-5-yl)methyl group and a (1,3-dixan-2-yl)methyl group, and a preferable example is a (1,3-dixan-5-yl)methyl group.

The term "anxiety" used in the present specification means not only anxiety in the strict sense, but also to conditions within the general concept of anxiety, such as generalized anxiety disorder, panic disorder, phobia, obsessive compulsive disorder and post-traumatic stress disorder, as well as diseases closely related to anxiety.

The term "dementia" used in the present specification means not only dementia in the strict sense, but also conditions within the general concept of dementia, such as Alzheimer-type senile dementia, multi-infarct dementia and senile dementia, as well as diseases closely related to dementia.

A "pharmacologically acceptable salt" used in the present specification is not particularly limited as long as it is formed with the compound of the present invention, and as specific examples thereof may include inorganic acid salts, organic acid salts, and acidic amino acid salts.

A "pharmacologically acceptable salt" used in the present specification, unless otherwise specified, may form a salt with an appropriate ratio, and the number of the acid molecule to one molecule of the compound is not particularly limited in the formed salt, but preferably about 0.1 to about 5 molecules of the acid exists with respect to one molecule of the compound, more preferably approximately 0.5 to approximately 2 molecules of the acid exists with respect to one molecule of the compound, and further preferably about 0.5, about 1 or about 2 molecules of the acid exists with respect to one molecule of the compound.

Preferable examples of inorganic acid salts may include hydrochloride, hydrobromide, sulfate, nitrate, phosphate and the like; preferable examples of organic acid salts may include acetate, succinate, fumarate, maleate, tartrate, citrate, lactate, stearate, benzoate, methanesulfonate, p-toluenesulfonate and the like.

Preferable examples of acidic amino acid salts may include aspartate, glutamate and the like.

In the present specification, R¹ in the formula (I) and R^{1x} in the formula (Ia) are preferably a C1-6 alkyl group, a C3-6 cycloalkyl group or a C3-6 cycloalkyl-C1-6 alkyl group and more preferably a C1-6 alkyl group, a cyclopropyl group or a cyclopropylmethyl group.

In the present specification, R² in the formula (I) and R^{2x} in the formula (Ia) are preferably (a) a cyclic group selected from a tetrahydropyranyl group, a dihydropyranyl group, a tetrahydrofuryl group and a dioxanyl group or (b) a C1-6 alkyl group substituted with a cyclic group selected from a tetrahydropyranyl group, a dihydropyranyl group, a tetrahydrofuryl group and a dioxanyl group and more preferably a tetrahydropyranyl group, a tetrahydropyranylmethyl group, a dioxanylmethyl group or a tetrahydrofurylmethyl group.

In the present specification, R³ in the formula (I) and R^{3x} in the formula (Ia) are preferably a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom, more preferably a halogen atom, methoxy or a C1-6 alkoxymethyl group, and even more preferably a C1-6 alkoxymethyl group.

In the present specification, R⁴ in the formula (I) and R^{4x} in the formula (Ia) are preferably a hydrogen atom, a C1-6 alkyl group or a C1-6 alkoxy group and more preferably a hydrogen atom or methoxy.

In the present specification, R⁵ in the formula (I) and R^{5x} in the formula (Ia) are preferably a halogen atom, a C1-6 alkyl group or a C1-6 alkoxy group and more preferably a halogen atom or methoxy.

In the present specification, R⁶ in the formula (I) and R^{6x} in the formula (Ia) are preferably a hydrogen atom or a methyl group.

In the present specification, R⁷ in the formula (I) and R^{7x} in the formula (Ia) are preferably a methyl group or an ethyl group.

### (General Production Process)

Hereinafter, General Production Processes of compounds according to the present application are shown, but they are not intended to be limited to these processes. Furthermore, the raw material compounds and reagents used in the general production processes for compounds of the present invention may also form salts or solvates (especially hydrates).
"Room temperature" described below refers to a range from about 10°C to 35°C.

The compounds represented by the formula (I) of the present invention can be produced by the following production methods.

### <Production Method A>

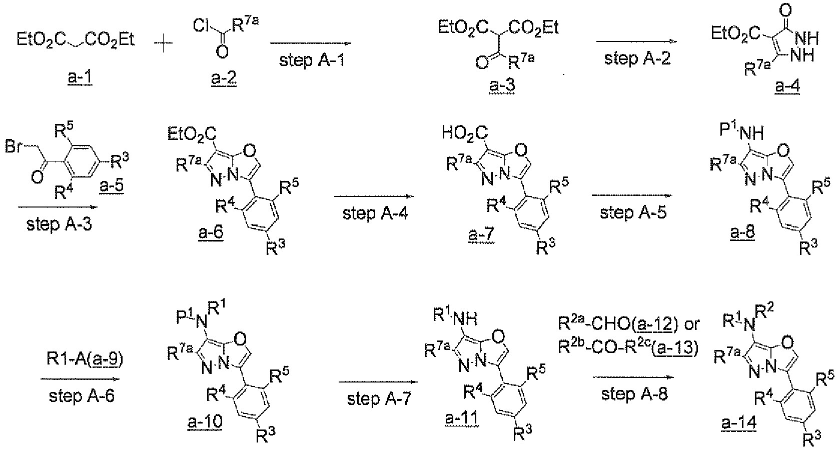

wherein R¹, R², R³, R⁴ and R⁵ have the same definitions as above, respectively; R^{7a} is a C1-6 alkyl group; P¹ is a protective group of an amino group; and A is a halogen atom. R^{2a}, R^{2b} and R^{2c} are groups where R^{2a}CH₂ and R^{2b}R^{2c}CH groups are R².

### <StepA-1>

This step is a step of treating diethyl malonate a-1 with magnesium (1 to 2 molar equivalents) in a solvent and reacting with Compound a-2 to yield Compound a-3.
This step can be specifically carried out according to the reaction conditions, the operations after reaction, and the purification method disclosed in Production Example 1-1 and the like described below.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent include alcohol solvents such as methanol and ethanol, ether solvents such as tetrahydrofuran, 1,2-dimethoxyethane, methyl-*tert*-butyl ether, cyclopentyl methyl ether, diethyl ether, diisopropyl ether, dibutyl ether, dicyclopentyl ether and 1,2-dimethoxyethane, aliphatic hydrocarbon solvents such as heptane and hexane or halogenated hydrocarbon solvents such as carbon tetrachloride, or mixed solvents thereof, and preferably a mixed solvent of ethanol and carbon tetrachloride.

Compound a-2 is preferably used at 0.9 to 1.2 molar equivalents with respect to Compound a-1.

The reaction temperature is usually different depending on the starting materials, the solvent, and the other reagents used in the reaction, and preferably is 0°C to the reflux temperature of the solvent (the internal temperature of the reaction apparatus).

The reaction time is usually different depending on the starting materials, the solvent, the other reagents used in the reaction, and the reaction temperature, and preferably is 0.5 to 5 hours at the above temperature after the addition of the reagents.

### <StepA-2>

This step is a step of reacting Compound a-3 with hydrazine hydrochloride (1 to 2 molar equivalents) in a solvent to yield Compound a-4.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent used include alcohol solvents such as methanol and ethanol, ether solvents such as tetrahydrofuran, 1,2-dimethoxyethane, methyl-*tert-*butyl ether, cyclopentyl methyl ether, diethyl ether, diisopropyl ether, dibutyl ether, dicyclopentyl ether, and 1,2-dimethoxyethane, or aromatic hydrocarbon solvents such as benzene and toluene, or mixed solvents thereof, and preferably ethanol.

The reaction temperature is usually different depending on the starting materials, the solvent, and the other reagents used in the reaction, and preferably is 0°C to the reflux temperature of the solvent (the internal temperature of the reaction apparatus).

The reaction time is usually different depending on the starting materials, the solvent, the other reagents used in the reaction, and the reaction temperature, and preferably is 1 to 24 hours at the above temperature after the addition of the reagents.

<Step A-3> This step is a step of reacting Compound a-4 with Compound a-5 in *N,N*-dimethylformamide in the presence of a base, then treating with an acid in a solvent to yield Compound a-6.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent used include alcohol solvents such as methanol and ethanol, ether solvents such as tetrahydrofuran, 1,2-dimethoxyethane, methyl-*tert*-butyl ether, cyclopentyl methyl ether, diethyl ether, diisopropyl ether, dibutyl ether, dicyclopentyl ether, and 1,2-dimethoxyethane, or aromatic hydrocarbon solvents such as benzene and toluene, acetic acid, or mixed solvents thereof

The base is different depending on the starting materials, the solvent used, and the like; examples of the base include, but are not particularly limited to, inorganic bases such as lithium carbonate, sodium carbonate, potassium carbonate, sodium hydrogen carbonate, potassium hydrogen carbonate, and cesium carbonate, and organic bases such as imidazole, pyridine, triethylamine, and *N,N*-diisopropylethylamine, and preferably potassium carbonate and the like. Then, 1 to 3 molar equivalents of the base with respect to Compound a-4 can be used, and preferred are 1 to 1.5 molar equivalents.

The acid used is different depending on the starting materials, the solvent used, and the like; examples of the acid include, but are not particularly limited to, acetic acid, hydrochloric acid, sulfuric acid, methanesulfonic acid, and *p*-toluenesulfonic acid, etc. Preferred is *p*-toluenesulfonic acid. Then, 1 to 3 molar equivalents of the acid with respect to Compound a-4 can be used, and preferred are 1 to 1.5 molar equivalents.

The reaction temperature is usually different depending on the starting materials, the solvent, and the other reagents used in the reaction, and preferably is 0°C to the reflux temperature of the solvent (the internal temperature inside of the reaction apparatus).

The reaction time is usually different depending on the starting materials, the solvent, the other reagents used in the reaction, and the reaction temperature, and preferably is 1 to 24 hours at the above temperature after the addition of the reagents.

### <StepA-4>

This step is a step of hydrolyzing Compound a-6 in a solvent to yield Compound a-7, and specifically can be carried out according to Production Example 1-4 described below.

The solvent used in this reaction is not particularly limited as long as it dissolves the starting materials to some extent and does not inhibit the reaction; examples of the solvent include ether solvents such as tetrahydrofuran and 1,4-dioxane and alcohol solvents such as methanol and ethanol, preferably the alcohol solvents, and more preferably ethanol.

As the reagent used in the hydrolysis, a reagent usually used in hydrolysis of ester can be used; examples of the reagent include, but are not particularly limited to, preferably 5 to 50 volumes of 2 N sodium hydroxide aqueous solution or 2 N potassium hydroxide aqueous solution.

The reaction temperature is usually different depending on the starting materials, the solvent, and the other reagents used in the reaction, preferably is 0°C to the reflux temperature of the solvent (the internal temperature of the reaction apparatus), and more preferably is room temperature to 80°C.

The reaction time is usually different depending on the starting materials, the solvent, the other reagents used in the reaction, and the reaction temperature, preferably is 1 to 12 hours at the above temperature after the addition of the reagents, and more preferably is 1 to 3 hours.

### <StepA-5>

This step is a step of reacting Compound a-7 via an acid azide derivative in the presence of a base with *tert*-butanol and performing a rearrangement reaction such as Curtius rearrangement to yield Compound a-8.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent preferably include benzene, toluene, xylene, diphenyl ether, *tert*-butanol, tetrahydrofuran, dioxane, acetonitrile, and *N, N-*dimethylformamide, and these can be used alone or mixed therewith.

The base is different depending on the starting materials, the reagents, and the like; examples of the base preferably include, but are not particularly limited to, triethylamine, *N*, *N*-diisopropylethylamine, 4-(dimethylamino) pyridine, and pyridine, and these can be used alone or mixed therewith. Then, 1 to 3 molar equivalents of the base with respect to Compound a-7 can be used, and preferred are 1 to 2 molar equivalents.

The azidation agent is different depending on the starting materials, the reagents, and the like; examples of the agent preferably include, but are not particularly limited to, and diphenylphosphoryl azide (DPPA). Then, 1 to 3 molar equivalents of the azidation agent with respect to Compound a-7 can be used, and preferred are 1 to 1.5 molar equivalents.

The reaction temperature is usually different depending on the starting materials, the solvent, and the other reagents used in the reaction, usually is -10°C to 250°C, and preferably is 100°C to 200°C.

The reaction time is usually different depending on the starting materials, the solvent, the other reagents used in the reaction, and the reaction temperature, preferably is 1 to 24 hours at the above temperature after the addition of the reagents, and preferably is 1 to 10 hours.

Alternatively, in order to synthesize the above acid azide derivative, a carboxylic acid derivative can be induced into an acid chloride or a mixed acid anhydride followed by reacting with an azidation agent (e.g., sodium azide, trimethylsilyl azide) to yield an acid azide derivative. In this case, 1 to 3 equivalents of the azidation agent, 1 to 5 equivalents of a base, and 1 to 50 equivalents or a solvent of *tert*-butanol can be desirably used. Further, alternatively, Compound a-8 can be obtained by Hoffmann rearrangement or Schmidt rearrangement.

### <Step A-6>

This step is a step of reacting Compound a-8 with Compound a-9, a halogenated alkyl, in a solvent in the presence of a base to yield Compound a-10.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent which can be used include nitrile solvents such as acetonitrile, ether solvents such as tetrahydrofuran, 1,2-dimethoxyethane, methyl-*tert*-butyl ether, cyclopentyl methyl ether, diethyl ether, diisopropyl ether, dibutyl ether, and dicyclopentyl ether, aromatic hydrocarbon solvents such as benzene and toluene, amide solvents such as *N,N*-dimethylformamide, sulfoxide solvents such as dimethyl sulfoxide, or aliphatic hydrocarbon solvents such as heptane and hexane, or mixed solvents thereof, and preferably the amide solvents, and more preferably *N,N*-dimethylformamide.

The base is different depending on the starting materials, the solvent used, and the like; examples of the solvent include, but are not particularly limited to, inorganic bases such as lithium hydroxide, sodium hydroxide, potassium hydroxide, lithium carbonate, sodium carbonate, potassium carbonate, sodium hydrogen carbonate, potassium hydrogen carbonate, cesium carbonate, and potassium-*tert*-butoxide, organic metal bases such as butyllithium, methyllithium, lithium bistrimethylsilylamide, sodium bistrimethylsilylamide, potassium bistrimethylsilylamide, metal hydride bases such as lithium hydride, sodium hydride, and potassium hydride, and organic bases such as imidazole, pyridine, 4-dimethylaminopyridine, triethylamine, and *N,N*-diisopropylethylamine, and preferably sodium hydride or sodium hydroxide. Then, 1 to 3 molar equivalents of the base with respect to Compound a-8 can be used, and preferred are 1 to 2 molar equivalents.

Then, 1 to 5 molar equivalents of Compound a-9 with respect to Compound a-8 can be used, and preferred is 1 to 2 molar equivalents.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature.

The reaction time is usually different depending on the starting materials, the solvent, the other reagents used in the reaction, and the reaction temperature, preferably is 0.5 to 12 hours at the above temperature after the addition of the reagents, and more preferably is 0.5 to 2 hours.

### <Step A-7>

This step is a step of deprotecting the protective group of an amino group of Compound a-10 to yield Compound a-11.
The deprotecting reaction of the protective group of the amino group is different depending on a type of the protective group and the like, and the deprotection can be performed, but is not particularly limited to, under an acidic condition for a *t*-butoxycarbonyl group and the like, for example.

Examples of the acid used in this reaction include trifluoroacetic acid, hydrochloric acid, sulfuric acid, and preferably trifluoroacetic acid. Then, 1 to 100 volumes of the acid with respect to Compound a-10 can be used.

The solvent used in this reaction is not particularly limited as long as it dissolves the starting materials to some extent and does not inhibit the reaction; examples of the solvent include halogenated hydrocarbon solvents such as dichloromethane, alcohol solvents such as methanol and ethanol, ether solvents such as tetrahydrofuran, 1,2-dimethoxyethane, methyl-*tert-*butyl ether, cyclopentyl methyl ether, diethyl ether, diisopropyl ether, dibutyl ether, and dicyclopentyl ether, or acetic acid, etc., and these can be used alone or as a mixed solvent.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature.

The reaction time is usually different depending on the starting materials, the solvent, the other reagents used in the reaction, and the reaction temperature, preferably is 1 to 48 hours at the above temperature after the addition of the reagents, and more preferably is 1 to 6 hours.

### <Step A-8>

This step is a step of carrying out a reductive amination reaction of Compound a-11 with Compound a-12 or Compound a-13, an aldehyde or ketone corresponding to R², in the presence of a reducing agent to yield Compound a-14.

The reducing agent can use a reducing agent that can be usually used in a reductive amination reaction of a carbonyl compound with an amine compound; examples of the reducing agent include, but are not limited in this reaction to, borane, and boron hydride complex compounds, etc., and preferably α-picoline borane or sodium triacetoxyborohydride. Then, 0.5 to 3 molar equivalents of the reducing agent with respect to Compound a-11 can be used, and preferred are 1 to 2 molar equivalents.

Then, 1 to 5 molar equivalents of Compound a-12 or Compound a-13 with respect to Compound a-11 can be used, and preferred are 1 to 2 molar equivalents.

The solvent used in this reaction is not particularly limited as long as it dissolves the starting materials to some extent and does not inhibit the reaction; examples of the solvent include alcohol solvents such as methanol and ethanol, ether solvents such as tetrahydrofuran, 1,2-dimethoxyethane, methyl-*tert*-butyl ether, cyclopentyl methyl ether, diethyl ether, diisopropyl ether, dibutyl ether, and dicyclopentyl ether, or acetic acid, etc., and these can be used alone or as a mixed solvent.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature.

The reaction time is usually different depending on the starting materials, the solvent, the other reagents used in the reaction, and the reaction temperature, preferably is 0.5 to 12 hours at the above temperature after the addition of the reagents, and more preferably is 1 to 6 hours.

### <Production Method B>

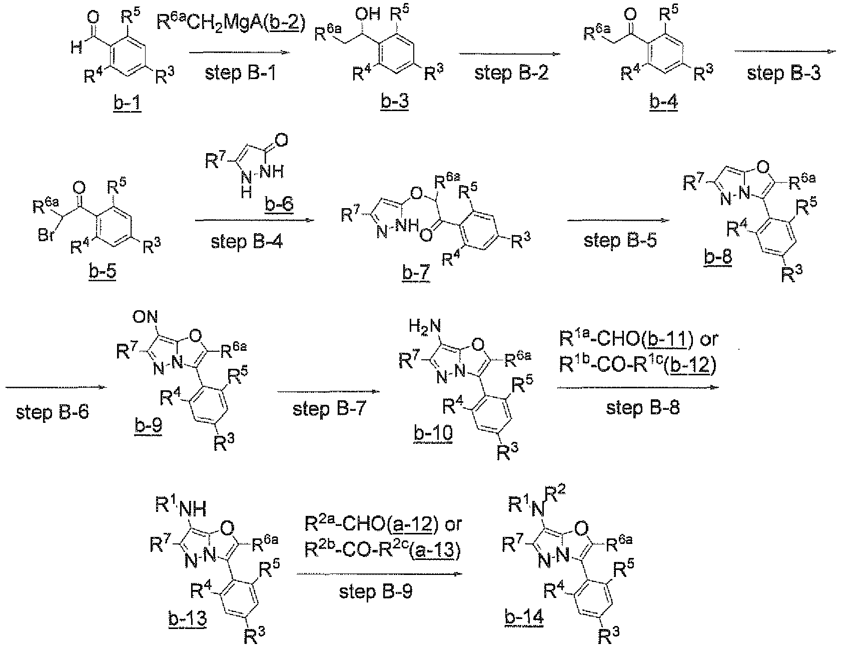

wherein R¹, R², R³, R⁴, R⁵, R⁷, A, R^{2a}, R^{2b} and R^{2c} have the same definitions as above, respectively; R^{6a} is a hydrogen atom or a C1-6 alkyl group; and R^{1a}, R^{1b} and R^{1c} are groups where R^{1a}CH₂ and R^{1b}R^{1c}CH groups are R².

### <Step B-1>

This step is a step of treating Compound b-1 with Grignard reagent b-2 (1 to 2 molar equivalents) in a solvent to yield Compound b-3.
This step can be specifically carried out according to the reaction conditions, the operations after reaction, and the purification method disclosed in Example 4a and the like described below.
This reaction may be carried out in a stream or in an atmosphere of an inert gas such as nitrogen or argon.

The solvent used in this reaction is not particularly limited as long as it dissolves the starting materials to some extent and does not inhibit the reaction: examples of the solvent include ether solvents such as tetrahydrofuran, 1,2-dimethoxyethane, methyl-*tert*-butyl ether, cyclopentyl methyl ether, diethyl ether, diisopropyl ether, dibutyl ether, dicyclopentyl ether, and 1,2-dimethoxyethane. Preferred is tetrahydrofuran or diethyl ether.

The reaction temperature is usually different depending on the starting materials, the solvent, and the other reagents used in the reaction, and preferably is 0°C to room temperature (the internal temperature inside of the reaction apparatus).

The reaction time is usually different depending on the starting materials, the solvent, the other reagents used in the reaction, and the reaction temperature, and preferably is 0.5 to 5 hours at the above temperature after the addition of the reagents.

### <Step B-2>

This step is a step of treating Compound b-3 with an oxidizing agent in a solvent to yield Compound b-4.

This step can be specifically carried out according to the reaction conditions, the operations after reaction, and the purification method disclosed in Example 4a and the like described below.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent preferably include acetone, dichloromethane, n-hexane, toluene, xylene, acetonitrile, and water, etc., and these can be used alone or mixed therewith.

The oxidizing agent are different depending on the starting materials, the reagents, and the like; examples of the oxidizing agent preferably include, but are not particularly limited to, potassium permanganate, silver oxide, activated manganese dioxide, pyridinium dichromate, sodium chlorite, and 4-methylmorpholine-4-oxide (1 to 5% molar equivalents of a tetrapropylammonium perruthenate catalyst), etc., and these can be used alone or mixed therewith. Then, 1 to 3 molar equivalents of the oxidizing agent with respect to Compound b-3 can be used, and preferred are 1 to 2 molar equivalents.

The reaction temperature is usually different depending on the starting materials, the solvent, and the other reagents used in the reaction, usually is -10°C to 200°C, and preferably is room temperature.

The reaction time is usually different depending on the starting materials, the solvent, the other reagents used in the reaction, and the reaction temperature, and preferably is 0.5 to 24 hours at the above temperature after the addition of the reagents.

### <Step B-3>

This step is a step of silylating Compound b-4 with *tert*-butyldimethylsilyl trifluoromethanesulfonate (1 to 2 molar equivalents) in a solvent in the presence of a base followed by bromination and a reaction by a desilylating agent to yield Compound b-5.

The solvent used in this silylation reaction is not particularly limited as long as it dissolves the starting materials to some extent and does not inhibit the reaction; examples of the solvent include tetrahydrofuran, diethyl ether, 1,4-dioxane, hexane, and pentane, etc., and these can be used alone or as a mixed solvent.

The base is different depending on the starting materials, the solvent used, and the like; examples of the solvent include, but are not particularly limited to, inorganic bases such as lithium hydroxide, sodium hydroxide, potassium hydroxide, lithium carbonate, sodium carbonate, potassium carbonate, sodium hydrogen carbonate, potassium hydrogen carbonate, and cesium carbonate, or organic bases such as imidazole, pyridine, triethylamine, and *N,N*-diisopropylethylamine, and preferably triethylamine and the like. In this case, 3 to 5 molar equivalents of the base with respect to Compound b-4 are preferably used.

The reaction temperature is usually different depending on the starting materials, the solvent, and the other reagents used in the reaction, preferably is 0°C to the reflux temperature of the solvent (the internal temperature inside of the reaction apparatus), and more preferably is 0°C to room temperature.

The reaction time is usually different depending on the starting materials, the solvent, the other reagents used in the reaction, and the reaction temperature, and preferably is 0.1 to 12 hours at the above temperature after the addition of the reagents.

The bromination reaction is usually carried out by one-pot approach after the silylation reaction. The brominating agent is different depending on the starting materials, the solvent used, and the like; examples of the agent include, but are not particularly limited to, bromine, N-bromosuccinimide, 1,2-dibromoethane, and 1,2-dibromo-1,1,2,2-tetrafluoroethane, etc. In this case, 1 to 2 molar equivalents of the brominating agent with respect to Compound b-4 is preferably used.

The reaction temperature is usually different depending on the starting materials, the solvent, and the other reagents used in the reaction, and preferably is 0°C to the reflux temperature of the solvent (the internal temperature inside of the reaction apparatus), and more preferably is 0°C to room temperature.

The reaction time is usually different depending on the starting materials, the solvent, the other reagents used in the reaction, and the reaction temperature, and preferably is 0.5 to 24 hours at the above temperature after the addition of the reagents.

The solvent used in the desilylation reaction is not particularly limited as long as it dissolves the starting materials to some extent and does not inhibit the reaction; examples of the solvent include tetrahydrofuran, diethyl ether, 1,4-dioxane, hexane, and pentane, etc., and these can be used alone or as a mixed solvent.

The desilylating agent is different depending on the starting materials, the solvent used, and the like; examples of the agent include, but are not particularly limited to, hydrogen fluoride, cesium fluoride, and tetrabutylammonium fluoride, etc., and preferably tetrabutylammonium fluoride. In this case, 0.5 to 1 molar equivalent of the desilylating agent with respect to Compound b-4 is preferably used.

The reaction temperature is usually different depending on the starting materials, the solvent, and the other reagents used in the reaction, and preferably is 0°C to the reflux temperature of the solvent (the internal temperature inside of the reaction apparatus), and more preferably is 0°C to room temperature.

The reaction time is usually different depending on the starting materials, the solvent, the other reagents used in the reaction, and the reaction temperature, and preferably is 0.1 to 12 hours at the above temperature after the addition of the reagents.

### <Step B-4>

This step is a step of reacting Compound b-5 with Compound b-6 in a solvent in the presence of a base to yield Compound b-7.

The solvent used in this reaction is not particularly limited as long as it dissolves the starting materials to some extent and does not inhibit the reaction; examples of the solvent include ether solvents such as tetrahydrofuran and 1,4-dioxane, or amide solvents such as *N,N-*dimethylformamide and *N-*methylpyrrolidone, and preferably the amide solvents, and more preferably *N,N*-dimethylformamide.

The base is different depending on the starting materials, and the solvent used; desirable examples of the base include, but are not particularly limited to, cesium carbonate, potassium carbonate, and sodium carbonate. Then, 2 to 10 molar equivalents of the base with respect to Compound b-5 can be used, and preferred are 2 to 4 molar equivalents.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature to 80°C.

The reaction time is usually different depending on the starting materials, the solvent, the other reagents used in the reaction, and the reaction temperature, preferably is 1 to 24 hours at the above temperature after the addition of the reagents, and more preferably is 2 to 10 hours.

### <Step B-5>

This step is a step of treating Compound b-7 with a microwave in a solvent in the presence of an acid to yield Compound b-8.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent which can be used include alcohol solvents such as methanol and ethanol, ether solvents such as tetrahydrofuran, 1,2-dimethoxyethane, methyl-tert-butyl ether, cyclopentyl methyl ether, diethyl ether, diisopropyl ether, dibutyl ether, dicyclopentyl ether, and 1,2-dimethoxyethane, aromatic hydrocarbon solvents such as benzene and toluene, or acetic acid, or mixed solvents thereof.

The acid used is different depending on the starting materials, the solvent used, and the like; examples of the acid include, but are not particularly limited to, acetic acid, hydrochloric acid, sulfuric acid, methanesulfonic acid, and *p*-toluenesulfonic acid, etc. Preferred is p-toluenesulfonic acid. Then, 1 to 3 molar equivalents of the acid with respect to Compound b-7 can be used.

The reaction temperature is usually different depending on the starting materials, the solvent, and the other reagents used in the reaction, and preferably is 0°C to 200°C.

The reaction time is usually different depending on the starting materials, the solvent, the other reagents used in the reaction, and the reaction temperature, and preferably is 1 to 24 hours at the above temperature after the addition of the reagents.

### <Step B-6>

This step is a step of nitrosating Compound b-8 in a solvent to yield Compound b-9.

The reaction conditions can use reaction conditions of typical nitrosation of a naromatic ring compound; for example, the reaction of 1 to 5 molar equivalents of sodium nitrite or potassium nitrite is preferably performed in an aqueous solvent in the presence of 10 to 100 molar equivalents of hydrochloric acid or sulfuric acid.

The reaction temperature is usually different depending on the starting materials, the solvent, and the other reagents used in the reaction, preferably is 0°C to the reflux temperature of the solvent (the internal temperature inside of the reaction apparatus), and more preferably is 0°C to room temperature.

The reaction time is usually different depending on the starting materials, the solvent, the other reagents used in the reaction, and the reaction temperature, preferably is 0.1 to 3 hours at the above temperature after the addition of the reagents, and more preferably is 0.5 to 1 hour.

### <Step B-7>

This step is a step of reducing Compound b-9 to yield Compound b-10.

Compound b-9 can be reduced in the presence of 1 to 10 molar equivalents of zinc, iron, tin(II) chloride, and nickel(II) chloride and 1 to 20 molar equivalents of an acid. The acid used is different depending on the starting materials, the solvent used, and the like; examples of the acid preferably include, but are not particularly limited to, acetic acid, hydrochloric acid, and sulfuric acid, etc.

The compound can also be reduced by hydrogen in the presence of a catalyst such as palladium-carbon. Then, 5 to 50% by weight of the catalyst with respect to Compound b-9 are preferably used.

The solvent used is different depending on the starting materials, the solvent used, and the like; examples of the solvent preferably include, but are not particularly limited to, methanol, ethanol, n-butanol, ethyl acetate, or water, etc., and these can be used alone or as a mixed solvent.

The reaction temperature is usually different depending on the starting materials, the solvent, and the other reagents used in the reaction, and usually is 0°C to room temperature.

The reaction time is usually different depending on the starting materials, the solvent, the other reagents used in the reaction, and the reaction temperature, and preferably is 1 to 24 hours at the above temperature after the addition of the reagents.

### <Step B-8>

This step is a step of treating Compound b-10 and Compound b-11 or Compound b-12, an aldehyde or ketone corresponding to R¹, respectively, with a reducing agent, in a solvent to yield Compound b-13.
This reaction may be carried out in a stream or in an atmosphere of an inert gas such as nitrogen or argon.

The solvent used in this reaction is not particularly limited as long as it dissolves the starting materials to some extent and does not inhibit the reaction; examples of the solvent include alcohol solvents such as methanol and ethanol, ether solvents such as tetrahydrofuran, 1,2-dimethoxyethane, methyl-*tert*-butyl ether, cyclopentyl methyl ether, diethyl ether, diisopropyl ether, dibutyl ether, and dicyclopentyl ether, or acetic acid, etc., and these can be used alone or as a mixed solvent

The reducing agent can use a reducing agent that can be usually used in the reductive amination reaction of a carbonyl compound with an amine compound; examples of the reducing agent include, but are not limited in this reaction to, borane, and boron hydride complex compounds, etc., and preferably α-picoline borane or sodium triacetoxyborohydride. In addition, 1.5 to 5 molar equivalents of the reducing agent with respect to the substrate can be used.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature.

The reaction time is usually different depending on the starting materials, the solvent, the other reagents used in the reaction, and the reaction temperature, preferably is 1 to 48 hours at the above temperature after the addition of the reagents, and more preferably is 1 to 10 hours.

### <Step B-9>

This step is a step of treating Compound b-13 and Compound a-12 or Compound a-13, an aldehyde or ketone corresponding to R², respectively, with a reducing agent in a solvent to yield Compound b-14. This step may be repeated by one-pot approach in a similar manner without isolating the product after step B-8. Alternatively, after isolating the product, the step may also be carried out in a manner similar to step A-8 of Production Method A.

### <Production Method C>

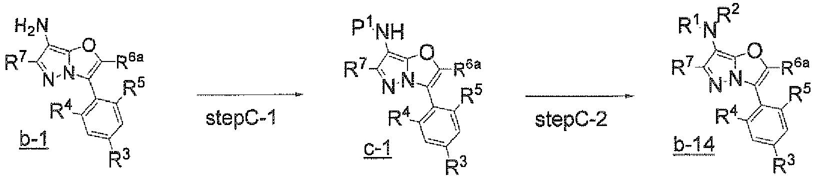

wherein R¹, R², R³, R⁴, R⁵, R⁷, R^{6a} and P¹ have the same definitions as above, respectively.
This Production Method C is an alternative for step B-8 and step B-9 in the Production Method B.

### <Step C-1>

This step is a step of reacting Compound b-1 with a protecting reagent for an amino group in a solvent in the presence or absence of a base to yield Compound c-1.
This step can employ a publicly-known reaction of introducing a protective group of an amino group, and specifically can be carried out according to a method of Example 14a described below.

The protecting reagent for an amino group can use publicly-known one; examples of the reagent include di-*tert*-butyl dicarbonate. Then, 1 to 1.5 molar equivalents of the reagent with respect to Compound c-1 can be used.

The solvent used in this reaction is not particularly limited as long as it dissolves the starting materials to some extent and does not inhibit the reaction; examples of the solvent include ether solvents such as tetrahydrofuran, diethyl ether, and 1,4-dioxane, halogenated hydrocarbon solvents such as dichloromethane, 1,2-dichloroethane, and chloroform, and amide solvents such as *N,N*-dimethylformamide, and preferably the halogenated hydrocarbon solvents, and more preferably tetrahydrofuran.

The base is different depending on the starting materials, the solvent used, and the like; examples of the solvent include, but are not particularly limited to, inorganic bases such as lithium hydroxide, sodium hydroxide, potassium hydroxide, lithium carbonate, sodium carbonate, potassium carbonate, sodium hydrogen carbonate, potassium hydrogen carbonate, and cesium carbonate, and organic bases such as imidazole, pyridine, triethylamine, and *N,N*-diisopropylethylamine, and preferably triethylamine and the like. Then, 1 to 2 molar equivalents of the base with respect to Compound c-1 can be used.

The reaction temperature is usually different depending on the starting materials, the solvent, and the other reagents used in the reaction, preferably is 0°C to the reflux temperature of the solvent (the internal temperature inside of the reaction apparatus), and more preferably is room temperature.

The reaction time is usually different depending on the starting materials, the solvent, the other reagents used in the reaction, and the reaction temperature, preferably is 1 to 48 hours at the above temperature after the addition of the reagents, and more preferably is 4 hours.

### <Step C-2>

This step is a step of alkylating the protected amino group of Compound c-1, deprotecting the protective group of the amino group, and performing a reductive amination reaction to yield Compound b-14. This step may be carried out in a method similar to step A-6, step A-7, and step A-8 in the Production Method A.

### [Reaction Treatment Method]

When the entire reaction mixture is a liquid, for example, the reaction mixture is returned to room temperature or cooled on ice as desired, and appropriately neutralized with an acid, an alkali, an oxidizing agent or a reducing agent, followed by addition of water and an organic solvent such as acetic acid which is immiscible with water and does not react with the target compound. After thoroughly shaking the mixture, the mixture is allowed to stand still and the layer containing the target compound is separated from the resulting two layers. Next, a solvent that is immiscible with the obtained layer and does not react with the target compound is added, and then the layer containing the target compound is washed and separated. When the layer is an organic layer, it may be dried with a desiccant such as anhydrous magnesium sulfate or anhydrous sodium sulfate, and the solvent is distilled off to yield the target compound When the layer is an aqueous layer, it is electrically desalted and then freeze-dried, and thereby the target compound can be obtained.

When the entire reaction mixture is a liquid, and if possible, it may be possible to collect the target compound simply by distilling off the components other than the target compound (for example, solvent, reagents, and the like) at ordinary pressure or under reduced pressure.

When only the target compound precipitates as a solid, or when the entire reaction mixture is a liquid and only the target compound precipitates as a solid during the collecting process, the target compound are first collected by a filtration method, the collected target compound are washed with a suitable organic or inorganic solvent and dried appropriately to yield the target compound.

Furthermore, when only the reagent or the catalyst is present as a solid, or when only the reagent or the catalyst precipitates as a solid during treatment of the reaction mixture, and the target compound is dissolved in a solution, the reagent or the catalyst is firstly removed by a filtration method, the removed reagent or catalyst is washed with a suitable organic or inorganic solvent, and the resultant washing solution is combined with the mother solution to yield a mixed solution, which is then treated in the same manner as in the case that the entire reaction mixture is a liquid, so that the target compound can be obtained.

The reaction mixture may be used directly for subsequent steps without isolation of the target compound in the case where components other than the target compound contained in the reaction mixture will not inhibit the reaction in the subsequent steps.

### [Purifying Method]

Purity of the target compound collected by the above-mentioned methods can be improved by appropriately carrying out recrystallization, various chromatography methods, or distillation.

When the collected target compound is a solid, purity of the target compound can be usually improved by recrystallization. For recrystallization, a simple solvent or a mixed solvent of a plurality of solvents, which does not react with the target compound, can be used. Specifically, firstly, the target compound is dissolved in the simple solvent or the mixed solvent of a plurality of solvents, which does not react with the target compound, at room temperature or with heating. The obtained mixture is cooled with ice water or the like or allowed to stand at room temperature to allow the target compound to precipitate from the mixed solution,

When the collected target compound is a solid or liquid, purity of the target compound can be improved by various chromatography methods. In general, a weakly acidic silica gel such as silica gel 60 (70-230 mesh or 340-400 mesh) by Merck, Ltd. or BW-300 (300 mesh) by Fuji Silysia Chemical, Ltd. may be used. If the target compound is basic, propylamine-coated silica gel (200-350 mesh) by Fuji Silysia Chemical, Ltd., or the like, may be used. If the target compound is bipolar or requires elution with a highly polar solvent such as methanol, NAM-200H or NAM-300H by Nagara Science Co., Ltd. may be used. By using these silica gels, the target compound may be eluted in the simple solvent or the mixed solvent of a plurality of solvents, which does not react with the target compound, and the solvent is distilled off to yield the target compound with improved purity

When the collected target compound is a liquid, purity of the target compound can also be improved by distillation. The temperature and degree of reduced pressure are appropriately adjusted depending on the target compound, and the target compound can be obtained by an ordinary distillation method.

When a compound of the present invention is obtained in free form, it may be converted to an acceptable salt of the compound by an ordinary method.

On the contrary, when a compound of the present invention is obtained as a salt, it can be converted into the free form of the compound by an ordinary method.

Furthermore, various isomers (for example, geometric isomers, optical isomers, rotational isomers, stereoisomers, tautomers, and the like) obtained for compounds of the present invention can be purified and isolated using ordinary separation means such as, for example, recrystallization, a diastereomer salt method, enzymatic separation method, or various chromatography methods (for example, thin-layer chromatography, column chromatography, gas chromatography, etc.).

### [Formulation]

When a compound of the present invention is used as a medicine, the compound of the present invention is usually used after it is mixed and formulated with appropriate additives. However, this does not negate the use of the compounds of the present invention in bulk forms as a medicine.

As additives there may be mentioned excipients, binders, lubricants, disintegrators, coloring agents, taste correctives, emulsifiers, surfactants, dissolving aids, suspending agents, isotonizing agents, buffering agents, antiseptic agents, antioxidants, stabilizers, absorption accelerators and the like which are commonly used in medicines, and these may also be used in appropriate combinations as desired.
As examples of excipients there may be mentioned lactose, white soft sugar, glucose, corn starch, mannitol, sorbitol, starch, pregelatinized starch, dextrin, crystalline cellulose, light silicic anhydride, aluminum silicate, calcium silicate, magnesium aluminometasilicate, calcium hydrogenphosphate and the like.
As examples of binders there may be mentioned polyvinyl alcohol, methylcellulose, ethylcellulose, gum Arabic, tragacanth, gelatin, shellac, hydroxypropylmethylcellulose, hydroxypropylcellulose, carboxymethylcellulose sodium, polyvinylpyrrolidone, macrogol and the like.
As examples of lubricants there may be mentioned magnesium stearate, calcium stearate, sodium stearyl fumarate, talc, polyethylene glycol, colloidal silica and the like.
As examples of disintegrators there may be mentioned crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch, carboxymethyl starch sodium and the like.
As coloring agents there may be mentioned those approved for addition to pharmaceuticals, such as iron sesquioxide, yellow iron sesquioxide, calamine, caramel, β-carotene, titanium oxide, talc, riboflavin sodium phosphate, yellow aluminum lake and the like.
As taste correctives there may be mentioned cocoa powder, menthol, aromatic powders, peppermint oil, camphor, cinnamon powder and the like.
As emulsifying agents or surfactants there may be mentioned stearyl triethanolamine, sodium lauryl sulfate, lauryl aminopropionic acid, lecithin, glycerin monostearate, sucrose fatty acid esters, glycerin fatty acid esters and the like.
As dissolving aids there may be mentioned polyethylene glycol, propylene glycol, benzyl benzoate, methanol, cholesterol, triethanolamine, sodium carbonate, sodium citrate, polysorbate 80, nicotinamide and the like.
As suspending agents there may be mentioned the aforementioned surfactants, as well as hydrophilic polymers such as polyvinyl alcohol, polyvinylpyrrolidone, methylcellulose, hydroxymethylcellulose, hydroxyethylcellulose and hydroxypropylcellulose.
As isotonizing agents there may be mentioned glucose, sodium chloride, mannitol, sorbitol and the like.
As buffering agents there may be mentioned phosphate, acetate, carbonate and citrate buffering solutions.
As antiseptic agents there may be mentioned methylparaben, propylparaben, chlorobutanol, benzyl alcohol, phenethyl alcohol, dehydroacetic acid, sorbic acid and the like.
As antioxidants there may be mentioned sulfate, ascorbic acid, α-tocopherol and the like.
As stabilizers there may be mentioned those commonly used in drugs.
As absorption accelerators there may also be mentioned those commonly used in medicines.

As formulations there may be mentioned oral forms such as tablets, powders, granules, capsules, syrups, lozenges and inhalants; topical formulations such as suppositories, ointments, eye salves, tapes, eye drops, nose drops, ear drops, poultices, lotions, and the like; or injections.
The aforementioned oral forms may be formulated with appropriate combinations of the additives mentioned above. Their surfaces may also be coated if necessary.
The aforementioned topical formulations may be formulated with appropriate combinations of the additives mentioned above, and especially excipients, binders, taste connectives, emulsifiers, surfactants, dissolving aids, suspending agents, isotonizing agents, antiseptic agents, antioxidants, stabilizers and absorption accelerators.
Injections may also be formulated with appropriate combinations of the additives mentioned above, and especially emulsifiers, surfactants, dissolving aids, suspending agents, isotonizing agents, buffering agents, antiseptic agents, antioxidants, stabilizers and absorption accelerators.

The dosage of a medicine according to the invention will differ depending on the severity of symptoms, patient age, gender and body weight, type of dosage form/salt, patient drug sensitivity and specific nature of the disease, but the dosage per day for adults will generally be 30 µg to 10 g (preferably 0.1 mg to 1 g) for oral administration, 30 µg to 20 g (preferably 100 µg to 10 g) for topical formulation and 30 µg to 1 g (preferably 100 µg to 1 g) for injection, either administered at a single time or divided into several dosages.
These values are the actual administered amounts in the case of oral formulations and injections, and are the amounts actually absorbed by the body in the case of topical formulations.

### [EXAMPLES]

The compounds of the present invention may be produced by the processes described in the following Examples, and the effects of the compounds may be confirmed by the methods described in the following testing examples. However, these specific examples are merely illustrative and not intended to limit the present invention in any way, while various modifications may be implemented within the scope of the present invention.

Compounds mentioned with reference to published documents were produced in the manner described in those documents.

The symbols used in the present specification stand for the followings.
¹H-NMR: proton nuclear magnetic resonance
δ: chemical shift
s: singlet
d: doublet
t: triplet
q: quartet
m: multiplet
dd: double doublet
br. s: broad singlet
sept: septet
J: coupling constant
Hz: Hertz
M: mol/L
n-: normal
s-: secondary
tert-: tertiary
N: normality
CDCl₃: deuterio-chloroform
EGTA: Glycol ether diamine tetraacetic acid (O,O'-bis(2-aminoethyl)ethyleneglycol-N,N,N,N-tetraacetic acid)
BSA: Bovine serum albumin
"Under reduced pressure" means conditions with approximately 1 to 50 mmHg by using a vacuum pump, a water-jet pump, and the like.

Unless otherwise specified, the "silica gel" in "silica, gel column chromatography" mentioned throughout the examples is Silica Gel 60 (70-230 mesh or 340-400 mesh) by Merck, Ltd., FLASH + Cartridge (KP-SIL, pore size: 60Å, particle size: 32-63 µm) by Biotage, or Cartridge (Hi-Flash, pore size: 60Å, particle size: 40 µm) by Yamazen.

Also unless otherwise specified, the "(NH)silica gel" in "(NH)silica gel column chromatography" mentioned throughout the examples is propylamine-coated silica gel (200-350 mesh) by Fuji Silysia Chemical, Ltd., or Cartridge (Hi-Flash Amino, pore size: 60Å, particle size: 40 µm) by Yamazen.

The term "room temperature" refers to a range from about 10°C to 35°C. The % denotes weight percent unless otherwise specified.

### [Production Example 1-1] diethyl acetylmalonate

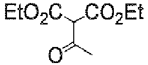

To a toluene solution (22 mL) of diethyl malonate (1.6 g, 10 mmol) were added magnesium turnings (243 mg, 10 mol), ethanol (1.93 mL, 33 mmol) and carbon tetrachloride (250 µL, 2.59 mmol) while stirring at room temperature, and the reaction mixture was stirred for 30 minutes at room temperature. After further heated under reflux for 1 hour at 85°C, this reaction mixture was cooled to 0°C, and to the reaction mixture was added acetyl chloride (714 µL, 10 mmol), and the reaction mixture was stirred for 30 minutes at 0°C. After the reaction mixture was returned to room temperature and stirred for 3 days, 5% hydrochloric acid was added thereto at 0°C. The organic layer was washed with a saturated sodium hydrogen carbonate aqueous solution and brine, dried over anhydrous magnesium sulfate, and thereafter the solvent was distilled off under reduced pressure. The residue obtained was dried to yield the title compound (1.54 g, 7.62 mmol).

### [Production Example 1-2] ethyl 5-methyl-3-oxo- 2,3-dihydro-1H-pyrazolo-4-carboxylate

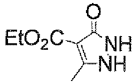

To an ethanol solution (20 mL) of diethyl acetylmalonate (2.99 g, 14.8 mmol) was added hydrazine monohydrochloride (1.52 g, 22.2 mmol), and the reaction mixture was heated under reflux for 10 hours at 85°C. The reaction mixture was returned to room temperature, and the solvent was distilled off under reduced pressure. To the residue obtained was added diethyl ether, and the resulting solid was filtered. After washed with a small amount of diethyl ether, the solid was dried to yield the title compound (0.74 g, 4.35 mmol).
¹H-NMR (CDCl₃) δ:1.39 (t, J=7.2 Hz, 3H), 2.48 (s, 3H), 4.37 (q, J=7.2 Hz, 2H).

### [Production Example 1-3] ethyl 3-(2,4-dichlorophenyl)-6-methylpyrazolo[5,1-b][1,3]oxazole-7-carboxylate

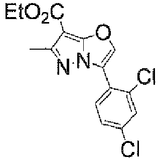

To an *N,N*-dimethylformamide solution (30 mL) of ethyl 5-methyl-3-oxo-2,3-dihydro-1H-pyrazolo-4-carboxylate (626 mg, 3.68 mmol) was added potassium carbonate (509 mg, 3.68 mmol), and the reaction mixture was stirred for 10 minutes at 50°C. To this reaction mixture was added an *N,N*-dimethylformamide solution (10 ml) of 2-bromo-2',4'-dichloroacetophenone (986 mg, 3.68 mmol), and the resulting reaction mixture was further stirred for 1 hour. The reaction mixture was added to water, and extracted with ethyl acetate, and then, the organic layer was washed with water and brine, dried over magnesium sulfate, and the solvent was distilled off under reduced pressure. The dried residue mixed with *p*-toluenesulfonic acid monohydrate (700 mg, 3.68 mmol), acetic acid (21 mL) and toluene (60 mL), and the mixture was heated under reflux for 5 hours at 120°C while removing water with a Dean-Stark apparatus. After the reaction mixture was returned to room temperature, the solvent was distilled off under reduced pressure. To the residue obtained was added ethyl acetate, the organic layer was washed with a saturated sodium hydrogen carbonate aqueous solution and brine, the organic layer was dried over magnesium sulfate, and then the solvent was distilled off under reduced pressure. To the residue was added a mixed solution of n-heptane and ethyl acetate (5:1), the resulting solid was filtered, and dried to yield the title compound (391 mg, 1.15 mmol).
¹H-NMR (CDCl₃) δ: 1.40 (t, J=7.2 Hz, 3H), 2.62 (s, 3H), 4.36 (q, J=7.2 Hz, 2H), 7.45 (dd, J=2.4, 8.4 Hz, 1H), 7.56 (d, J=2.4 Hz, 1H), 8.16 (s, 1H), 8.55 (d, J=8.4 Hz, 1H).

### [Production Example 1-4] 3-(2,4-dichlorophenyl)-6-methylpyrazolo[5,1-b][1,3]oxazole-7-carboxylic acid

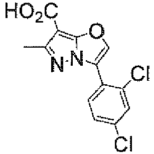

To an ethanol solution (5.75 mL) of ethyl 3-(2,4-dichlorophenyl)-6-methylpyrazolo[5,1-b][1,3]oxazole-7-carboxylate (391 mg, 1.15 mmol) was added 2 N sodium hydroxide (5.75 mL), and the reaction mixture was stirred for 1 hour at 60°C. The reaction mixture was further heated under reflux for 2.5 hours at 85°C. After the reaction mixture was returned to room temperature, the solvent was distilled off under reduced pressure. To the residue were added water and 2 N hydrochloric acid aqueous solution, which made the solution acidic. The resulting solid was filtered, and dried to yield the title compound (342 mg, 1.10 mmol).
¹H-NMR (CD₃OD) δ: 2.57 (s, 3H), 7.54 (dd, J=2.0, 8.4 Hz, 1H), 7.69 (d, J=2.0 Hz, 1H), 8.31 (s, 1H), 8.39 (d, J=8.4 Hz, 1H).

### [Production Example 1-5] tert-butyl [3-(2,4-dichlorophenyl)-6-methylpyrazolo[5,1-b][1,3]oxazol-7-yl]carbamate

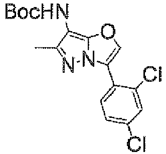

To a toluene solution (2.0 mL) of 3-(2,4-dichlorophenyl)-6-methylpyrazolo[5,1-b][1,3]oxazole-7-carboxylic acid (40.9 mg, 0.131 mmol) were added diphenylphosphoryl azide (31 µL, 0.144 mmol), triethylamine (21.9 µL, 0.157 mmol) and *tert*-butyl alcohol (240 µL, 2.62 mmol), and the reaction mixture was heated under reflux at 110°C for 1 hour and 20 minutes. After the reaction mixture was returned to room temperature, ethyl acetate was added thereto, the insoluble solid was filtered, and then the solvent was distilled off under reduced pressure. The residue was purified by medium-pressure silica-gel column chromatography (n-heptane/ ethyl acetate: 50%) to yield the title compound (32.5 mg, 0.085 mmol).
¹H-NMR (CDCl₃) δ: 1.46-1.58 (m, 9H), 2.34 (s, 3H), 5.76 (br.s, 1H), 7.37-7.46 (m, 1H), 7.54-7.56 (m, 1H), 8.04 (s, 1H), 8.55-8.58 (m, 1H).

### [Production Example 2-1]diethyl propionylmalonate

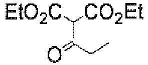

To a toluene solution (206 mL) of diethyl malonate (15 g, 93.8 mmol) were added magnesium turnings (2.28 g, 93.8 mol), ethanol (18.1 mL, 309 mmol) and carbon tetrachloride (2.34 mL, 24.2 mmol) while stirring at room temperature, and the reaction mixture was stirred for 30 minutes at room temperature. After further heated under reflux for 1 hour at 85°C, this reaction mixture was cooled to 0°C, and to the reaction mixture was added propionyl chloride (8.2 mL, 93.8 mmol), and the reaction mixture was stirred for 30 minutes at 0°C. After the reaction mixture was returned to room temperature and stirred for 3 days, 5% hydrochloric acid was added thereto at 0°C, the organic layer was washed with a saturated sodium hydrogen carbonate aqueous solution and brine, dried over anhydrous magnesium sulfate, and thereafter the solvent was distilled off under reduced pressure. The residue obtained was dried to yield the title compound (18.52 g, 85.6 mmol).

### [Production Example 2-2] ethyl 5-ethyl-3-oxo-2,3-dihydro-1H-pyrazolo-4-carboxylate

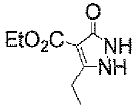

To an acetic acid solution (23 mL) of diethyl propionylmalonate (5 g, 23.1 mmol) was added hydrazine monohydrate (1.46 mL, 30 mmol) at 0°C, and the reaction mixture was heated under reflux for 3 hours at 120°C. The reaction mixture was returned to room temperature, and the solvent was distilled off under reduced pressure. To the residue obtained was added diethyl ether, and the resulting solid was filtered, the residue was washed with a small amount of diethyl ether, and the solid was then dried to yield the title compound (1.19 g, 6.46 mmol).
¹H-NMR (CDCl₃) δ: 1.28 (t, J=7.6 Hz, 3H), 1.38 (t, J=7.2 Hz, 3H), 2.87 (q, J=7.6 Hz, 2H), 4.36 (q, J=7.2 H_{z}, 2H).

### [Production Example 2-3] ethyl 3-(2,4-dimethoxyphenyl)-6(ethylpyrazolo[5,1-b][1,3]oxazole-7-carboxylate

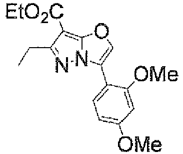

To an *N,N-*dimethylformamide solution (30 mL) of ethyl 5-ethyl-3-oxo-2,3-dihydro-1H-pyrazolo-4-carboxylate (500 mg, 2.71 mmol) was added potassium carbonate (375 mg, 2.71 mmol), and the reaction mixture was stirred for 10 minutes at 50°C. To this reaction mixture was added an *N,N-*dimethylformamide solution (10 mL) of 2-bromo-2',4'-dimethoxyacetophenone (702 mg, 2.71 mmol), and the resulting reaction mixture was further stirred for 1 hour. The reaction mixture was added to water, and extracted with ethyl acetate, and then, the organic layer was washed with water and brine, dried over magnesium sulfate, and thereafter the solvent was distilled off under reduced pressure. The dried residue was mixed with *p*-toluenesulfonic acid monohydrate (515 mg, 2.71 mmol), acetic acid (10.5 mL) and toluene (30 mL), and the mixture was heated under reflux for 4 hours at 120°C while removing water with a Dean-Stark apparatus. After the reaction mixture was returned to room temperature, the solvent was distilled off under reduced pressure. To the residue obtained was added ethyl acetate, and the organic layer was washed with a saturated sodium hydrogen carbonate aqueous solution and brine, After the organic layer was dried over magnesium sulfate, the solvent was distilled off under reduced pressure. The residue was purified by medium-pressure silica-gel column chromatography (n-heptane/ethyl acetate: 33% to 50%) to yield the title compound (359 mg, 1,04 mmol).
¹H-NMR (CDCl₃) δ: 1.34-1.42 (m, 6H), 3.05 (q, J=7.7 Hz, 2H), 3.88 (s, 3H), 3.96 (s, 3H), 4.36 (q, J=7.2 Hz, 2H), 6.58 (d, J=2.4 Hz, 1H), 6.70 (dd, J=2.4, 8.8 Hz, 1H), 7.99 (s, 1H), 8.80 (d, J=8.8 Hz, 1H.

### [Production Example 2-4] 3-(2,4-dimethoxyphenyl)-6-ethylpyrazolo[5,1-b][1,3]oxazole-7-carboxylic acid,

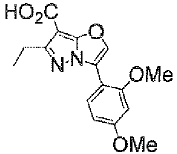

To an ethanol solution (5.20 mL) of ethyl 3-(2,4-dimethoxyphenyl)-6-ethylpyrazolo[5,1-b][1,3]oxazole-7-carboxylate (359 mg, 1.04 mmol) was added 2 N sodium hydroxide aqueous solution (5.20 mL), and the reaction mixture was stirred for 1 hour at 60°C. The reaction mixture was further heated under reflux for 2.5 hours at 85°C. After the reaction mixture was returned to room temperature, the solvent was distilled off under reduced pressure. To the residue were added water and 2 N hydrochloric acid, which made the solution acidic. After filtered and washed with a small amount of water, the resulting solid was dried to yield the title compound (263 mg, 0.83 mmol).
¹H-NMR (CD₃OD) δ: 1.34 (t, J=7.2 Hz, 3H), 3.01 (q, J=7.2 Hz, 2H), 3.88 (s, 3H), 3.99 (s, 3H), 6.70-6.73 (m, 2H), 8.19 (s, 1H), 8.69 (d, J=9.2 Hz, 1H).

### [Production Example 2-5] tert-butyl [3-(2,4-dimethoxyphenyl)-6-ethylpyrazolo[5,1-b][1,3]oxazol-7-yl]carbamate

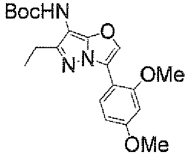

To a toluene solution (12.7 mL) of 3-(2,4-dimethoxyphenyl)-6-ethylpyrazolo[5,1-b][1,3]oxazole-7-carboxylic acid (263 mg, 0.831 mmol) were added diphenylphosphoryl azide (197 µL, 0.914 mmol), triethylamine (139 µL, 1.0 mmol) and *tert*-butyl alcohol (1.52 mL), 16.6 mmol), and the reaction mixture was heated under reflux at 110°C for 1 hour. After the reaction mixture was returned to room temperature, ethyl acetate was added thereto, the insoluble material was then filtered, and the solvent was distilled off under reduced pressure. The residue was purified by medium-pressure silica-gel column chromatography (n-heptane/ethyl acetate: 50%) to yield the title compound (133.1 mg, 0.34 mmol).
¹H-NMR (CDCl₃) δ:1.28-1.38(m, 3H), 1.40-1.50 (m, 9H), 2.68-2.78 (m, 2H), 3.86 (s, 3H), 3.94 (s, 3H), 6.54-6.60 (m, 1H), 6.64-6.72 (m, 1H), 7.87 (s, 1H), 8.80-8.88 (m, 1H).

### [Example 1] N-(cyclopropylmethyl)-3-(2,4-dichlorophenyl)-6-methyl-N-(tetrahydro-2H-pyran-ylmethy l)pyrazolo[5,1-b][1,3]oxazole-7-amine

### (1a) tert-butyl (cyclopropylmethyl)[3-(2,4-dichlorophenyl)-6-methylpyrazolo[5,1-b][1,3]oxazol-7-yl]carba mate

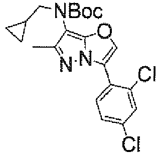

To an *N,N-*dimethylformamide solution (1.0 mL) of *tert*-butyl [3-(2,4-dichlorophenyl)-6-methylpyrazolo[5,1-b][1,3]oxazol-7-yl]carbamate (32.5 mg, 0.085 mmol) were added sodium hydride (4.08 mg, 0.102 mmol) and cyclopropylmethylbromide (16.5 µl, 0.17 mmol), and the reaction mixture was stirred for 1 hour at room temperature. To the reaction mixture was added water, and the reaction mixture was extracted with ethyl acetate, and then, the organic layer was washed with brine, dried over magnesium sulfate, and thereafter the solvent was distilled off under reduced pressure. The residue was purified by medium-pressure silica-gel column chromatography, (n-heptane/ethyl acetate: 50%) to yield the title compound (18.2 mg, 0.042 mmol).
¹H-NMR (CDCl₃) δ:0.12-0.20 (m, 2H), 0.38-0.52 (m, 2H), 0.70-1.61 (m, 10H), 2.33 (s, 3H), 3.34-3.50 (m, 2H), 7.42-7.50 (m, 1H), 7.52-7.59 (m, 1H), 8.05-8.16 (m, 1H), 8.66-8.78 (m, 1H).

### (1b) N-(cyclopropylmethyl)-3-(2,4-dichlorophenyl)-6-methyl-N-(tetrahydro-2H-pyran-4-ylmethy l)pyrazolo[5,1-b][1,3]oxazole-7-amine

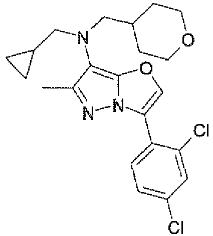

To a dichloromethane solution (1.0 mL) of *tert*-butyl (cyclopropylmethyl)[3-(2,4-dichlorophenyl)-6-methylpyrazolo[5,1-b][1,3]oxazol-7-yl]carba mate (77 mg, 0.176 mmol) was added trifluoroacetic acid (1.0 mL), and the reaction mixture was stirred for 1 hour at room temperature. Trifluoroacetic acid (1.0 ml) was further added thereto, the mixture was stirred for 40 minutes, and then, trifluoroacetic acid (0.5 mL) was again added, and the mixture was stirred for 30 minutes. The solvent was distilled off under reduced pressure, the resulting residue was dissolved in tetrahydrofuran (2.0 mL), and to this reaction mixture were added tetrahydro-2H-pyran-4-carbaldehyde (40.2 µL, 0.352 mmol) and sodium triacetoxyborohydride (56 mg, 0.264 mmol), and the mixture was stirred for 1 hour at room temperature. A saturated sodium hydrogen carbonate aqueous solution was added thereto, the mixture was extracted with ethyl acetate, and then, the organic layer was washed with brine, dried over magnesium sulfate, and the solvent was distilled off under reduced pressure. The residue was purified by medium-pressure silica-gel column chromatography (n-heptane/ethyl acetate: 20%) to yield the title compound (42.3 mg, 0.097 mmol).
¹H-NMR (CDCl₃) δ: -0.01-0.80 (m, 2H), 0.39-0.44 (m, 2H), 0.82-0.92 (m, 1H), 1.22-1.34 (m, 2H), 1.50-1.62 (m, 1H), 1.69-1.76 (m, 2H), 2.35 (s, 3H), 2.74 (d, J=6.4 Hz, 2H), 2.90 (d, J=7.6 Hz, 2H), 3.26-3.55 (m, 2H), 3.90-3,97 (m, 2H), 7.44 (dd, J=1.6, 8.4 Hz, 1H), 7.53 (d, J=1.6 Hz, 1H), 8.05 (s, 1H), 8.72 (d, J=8.4 Hz, 1H).

### [Example 2] N-cyclopropymethyl)-3-(2,4-dimethoxyphenyl)-6-ethyl-N-(tetrahydro-2H-pyran-4-ylmeth yl)pyrazolo[5,1-b][1,3]oxazole-7-amine

### 2a) tert-butyl (cyclopropylmethyl)[3-(2,4-dimethoxvphenyl)-6-ethylpyrazolo[5,1-b][1,3]oxazol-7-yl]carba mate

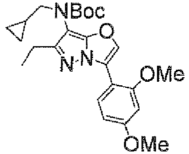

To an *N,N-*dimethylformamide solution (3.4 mL) of *tert*-butyl [3(2,4-dimethoxyphenyl)-6-ethylpyrazolo[5,1-b][1,3]oxazol-7-yl]carbamate (133 mg, 0.343 mmol) were added sodium hydride (16.5 mg, 0.412 mmol) and cyclopropylmethylbromide (66.5 µL, 0.686 mmol), and the reaction mixture was stirred for 30 minutes at room temperature. Further, sodium hydride (16.5 mg, 0.412 mmol) and cyclopropylmethylbromide (66.5 µL, 0.686 mmol) were added thereto, and the reaction mixture was stirred for 30 minutes at room temperature. To the reaction mixture was added water, and the reaction mixture was extracted with ethyl acetate, and then, the organic layer was washed with brine, dried over magnesium sulfate, and thereafter the solvent was distilled off under reduced pressure. The residue was purified by medium-pressure silica-gel column chromatography (n-heptane/ethyl acetate: 33%) to yield the title compound (62.9 mg, 0.14 mmol).
¹H-NMR (CDCl₃) δ: 0.12-0.20 (m, 2H), 0.40-0.48 (m, 2H), 0.98-1.08 (m, 1H), 1.24-1.60 (m, 12H), 2.71 (q, J=7.6 Hz, 2H), 3.32-3.48 (m, 2H), 3.88 (s, 3H), 3.96 (s, 3H), 6.58 (d, J=2.0 Hz, 1H), 6.71 (dd, J=2.0, 8.8 Hz, 1H), 7.90 (s, 1H), 8.90 (d, J=8.8 Hz, 1H).

### (2b) N-(cyclopropylmethyl)-3-(2,4-dimethoxyphenyl-6-ethyl-N-(tetrahdro-2H-pyran-4-ylmeth yl)pyrazolo[5,1-b][1,3]oxazole-7-amine

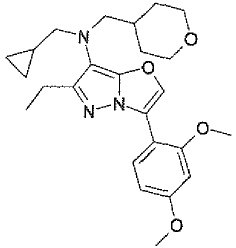

To a dichloromethane solution (2.0 mL) of *tert*-butyl (cyclopropylmethyl)[3-(2,4-dimethoxyphenyl)-6-ethylpyrazolo[5,1-b][1,3]oxazol-7-yl]carba mate (62.9 mg, 0.142 mmol) was added trifluoroacetic acid (1.0 mL), and the reaction mixture was stirred for 1 hour at room temperature. The solvent was distilled off under reduced pressure, the resulting residue was dissolved in tetrahydrofuran (2.0 mL), to the reaction mixture were added tetrahydro-2H-pyran-4-carbaldehyde (32.4 µL, 0.284 mmol) and sodium triacetoxyborohydride (45.1 mg, 0.213 mmol), and the reaction mixture was stirred for 1 hour at room temperature. To the reaction mixture was added a saturated sodium hydrogen carbonate aqueous solution, and the mixture was extracted with ethyl acetate, and then, the organic layer was washed with brine, dried over magnesium sulfate, and the solvent was distilled off under reduced pressure. The residue was purified by medium-pressure silica-gel column chromatography (n-heptane/ethyl acetate: 33%) to yield the title compound (25.5 mg, 0.058 mmol).
¹H-NMR (CDCl₃) δ: -0.02-0.08 (m, 2H), 0.37-0.44 (m, 2H), 0.82-0.94 (m, 1H), 1.21-1.38 (m, 2H), 1.34 (t, J=7.6 Hz, 3H), 1.49-1.65 (m, 1H), 1.68-1.77 (m, 2H), 2.72-2.80 (m, 4H), 2.89 (d, J=6.8 Hz, 2H), 3.25-3.35 (m, 2H), 3.84-3.98 (m, 2H), 3.86 (s, 3H), 3.94 (s, 3H), 6.55 (d, J=2.4 Hz, 1H), 6.69 (dd, J=2.4, 8.8 Hz, 1H), 7.85 (s, 1H), 8.89 (d, J=8.8 Hz, 1H).

### [Example 3] N-(cyclopropylmethyl)-3-[2,6-dimethoxy-4-(methoxymethyl)phenyl]-6-ethyl-N-(tetrahydro-2H-pyran-4-ylmethyl)pyrazolo[5,1-b][1,3]oxazole-7-amine

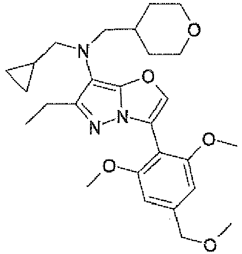

The compound of Example 3 was synthesized similarly to Example 2.
¹H-NMR (CDCl₃) δ: 0.02-0.07 (m, 2H), 0.39-0.45 (m, 2H), 0.86-0.96 (m, 1H), 1.20-1.35 (m, 6H), 1.72-1.80 (m, 2H), 2.68 (q, J=7.6 Hz, 2H), 2.74 (d, J=6.4 Hz, 2H), 2.90 (d, J=7.2 Hz, 2H), 3.30-3.39 (m, 2H), 3.43 (s, 3H), 3.82 (s, 6H), 3.91-3.99 (m, 2H), 4.48 (s, 2H), 6.63 (s, 2H), 7.3 (s,1H).

### [Example 4] 3-[2,6-dímethoxy-4-(methoxymethyl)phenyl]-6-ethyl-2-methylpyrazolo[5,1-b][1,3]oxazole-7-amine

### 4a 1-[2,6-dimethoxy-4-(methoxymethyl)phenyl]propan-1-one

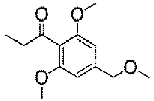

To a tetrahydrofuran solution (180 mL) of 2,6-dimethoxy-4-(methoxymethyl)benzaldehyde [CAS No. 114973-04-5] (Australian Journal of Chemistry (1987), 40 (11), 1841-50.) (15.6 g, 74.2 mmol) was added magnesium ethyl bromide (1 M, a tetrahydrofuran solution, 95 mL) while stirring under ice-cooling, and the mixture was stirred for 30 minutes at the same temperature. A saturated ammonium chloride aqueous solution was added thereto, the mixture was extracted with ethyl acetate, and then, the mixture was washed with brine, dried over magnesium sulfate, filtered, and thereafter the solvent was distilled off under reduced pressure to yield a crude product (17.9 g) of 1-[2,6-dimethoxy-4-(methoxymethyl)phenyl]propan-1-ol.
The resulting alcohol was dissolved in methylene chloride (135 mL) and acetonitrile (15 mL), and thereto Molecular sieves 4A (37 g), 4-methylmorpholine-4-oxide (13.5 g, 115 mmol) and tetrapropylammonium perruthenate (1.2 g, 3.4 mmol) were added at room temperature, and the reaction mixture was stirred for 15 hours at the same temperature. After ethyl acetate (50 ml) was added to the reaction mixture, the mixture was filtered through a small amount of silica gel, and the solvent of the filtrate was distilled off under reduced pressure.
The residue obtained was purified by medium-pressure silica-gel column chromatography (ethyl acetate/n-heptane: 10% to 30%) to yield the title compound (16.2 g, 68 mmol).
¹H-NMR (CDCl₃) δ: 1.14 (t, J=7.2 Hz, 3H), 2.74 (q, J=7.2 Hz, 2H), 3.40 (s, 3H), 3.78 (s, 6H), 4.43 (s, 2H), 6.52 (s, 2H).

### (4b) 2-bromo-1-[2,6-dimethoxy-4-(methoxymethyl)phenyl]propan-1-one

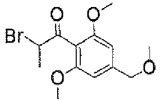

A tetrahydrofuran solution (170 mL) of 1-[2,6-dimethoxy-4-(methoxymethyl)phenyl]propan-1-one (16.2 g, 68 mmol) and triethylamine (28.5 mL, 205 mmol) was added tert-butyldimethylsilyl trifluoromethanesulfonate (23.4 mL, 102 mmol) under ice-cooling, and the mixture was then stirred at the same temperature for 20 minutes. *N*-bromosuccinimide (18.2 g, 102 mmol) was added slowly at 0°C and stirred for 5 minutes, and the mixture was stirred at room temperature for 1 hour. Then, *N*-bromosuccinimide (1.2 g, 6.8 mmol) was added three times every 10 minutes at room temperature, and thereafter, the mixture was stirred for 30 minutes at room temperature. A saturated sodium hydrogen carbonate aqueous solution was added thereto, the mixture was extracted with ethyl acetate, and then, the mixture was washed with brine, dried over magnesium sulfate, filtered, and thereafter, the solvent was distilled off under reduced pressure. The residue obtained was purified by medium-pressure silica-gel column chromatography (ethyl acetate/n-heptane: 10% to 25%) to yield a mixture of the title compound and enol silyl ether thereof.
To a tetrahydrofuran solution (70 mL) of the obtained mixture was added tetrabutylammonium fluoride (1 M, a tetrahydrofuran solution, 34 mL) at room temperature, and the resulting mixture was stirred for 10 minutes at the same temperature. A saturated ammonium chloride aqueous solution was added thereto, the mixture was extracted with ethyl acetate, and then, the mixture was washed with brine, dried over magnesium sulfate, filtered, and thereafter, the solvent was distilled off under reduced pressure. The residue was purified by medium-pressure silica-gel column chromatography (ethyl acetate/n-heptane: 10% to 30%) to yield the title compound (19.6 g, 61.8 mmol).
¹H-NMR (CDCl₃) δ: 1.84 (d, J=6.8 Hz, 3H), 3.42 (s, 3H), 3.83 (s, 6H), 4.45 (s, 2H), 5.03 (q, J=6.8 Hz, 1H), 6.56 (s, 2H).

### (4c) 1-[2,6-dimethoxy-4-(methoxymethyl)phenyl]-2-[(3-ethyl-1H-pyrazol-5-yl)oxy]propan-1-on e

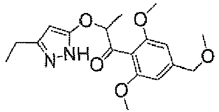

To an *N,N*-dimethylformamide solution (40 mL) of 2-bromo-1-[2,6-dimethoxy-4-(methoxymethyl)phenyl]propan-1-one (7 g, 22.1 mmol) and 3-ethyl-2-pyrazoline-5-one [CAS No. 110475-21-3] (WO2002/066439) (9.7 g, 86.5 mmol) was added cesium carbonate (14 g, 43 mmol) at room temperature, and the mixture was stirred for 2 hours at 80°C. A saturated ammonium chloride aqueous solution was added thereto under ice-cooling, the mixture was extracted twice with ethyl acetate, and then, the mixture was washed with brine, dried over magnesium sulfate, filtered, and thereafter, the solvent was distilled off under reduced pressure. The residue was purified by NH silica-gel column chromatography (ethyl acetate/n-heptane: 25% to 90%) to yield the title compound (4.6g, 13.2 mmol).
¹H-NMR (CDCl₃) δ: 1.22 (t, J=7.6 Hz, 3H), 1.55 (d, J=7.6 Hz, 3H), 2.57 (q, J=6.8 Hz, 2H), 3.41 (s, 3H), 3.77 (s, 6H), 4.43 (s, 2H), 5.47 (q, J=6.8 Hz, 1H), 5.51 (s, 1H), 6.52 (s, 2H).

### (4d) 3-[2,6-dimethoxy-4-(methoxymethyl)phenyl]-6-ethyl-2-methylpyrazolo[5,1-b][1,3]oxazole

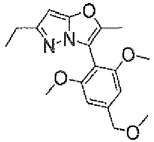

1-[2,6-Dimethoxy-4-(methoxymethyl)phenyl]-2-[(5-ethyl-1H-pyrazol-3-yl)oxy]pr opan-1-one (4.6 g, 13.2 mmol) and *p*-toluenesulfonic acid monohydrate (2.5 g, 13.1 mmol) were dissolved in acetic acid (34 mL), and the mixture was reacted in a microwave reactor (160°C, 3 bar) for 4 hours. After cooling, acetic acid was distilled off under reduced pressure below 40°C. The residue was purified by NH silica-gel column chromatography (ethyl acetate/n-heptane: 10% to 70%) to yield a mixture of the title compound and acetyl amide of the starting material (3.5 g).
The mixture obtained was dissolved in methanol (25 mL) and water (12 mL), potassium carbonate (2 g) was added thereto at room temperature, and the resulting mixture was stirred for 30 minutes at the same temperature. The solvent was distilled off under reduced pressure, and then brine was added thereto. After extracted with ethyl acetate, the mixture was washed with brine, dried over magnesium sulfate, filtered, and thereafter, the solvent was distilled off under reduced pressure. The residue was purified by silica-gel column chromatography (ethyl acetate/n-heptane: 10% to 100%) to yield the title compound (2.6 g, 7.9 mmol).
¹H-NMR (CDCl₃) δ: 1.24 (t, J=7.6 Hz, 3H), 2.20 (s, 3H), 2.69 (q, J=7.6 Hz, 2H), 3.44 (s, 3H), 3.79 (s, 6H), 4.48 (s, 2H), 5.61 (s, 1H), 6.62 (s, 2H).

### (4e) 3-[2,6-dimethoxy-4-(methoxymethyl)phenyl]-6-ethyl-2-methylpyrazolo[5,1-b][1,3]oxazole-7-amine

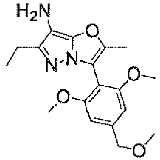

3-[2,6-Dimethoxy-4-(methoxymethyl)phenyl]-6-ethyl-2-methylpyrazolo[5,1-b][1, 3]oxazole (1 g, 3.0 mmol) was dissolved in hydrochloric acid (5 N, 24 mL), and a solution in which sodium nitrite (0.42 g, 6.1 mmol) was dissolved in water (6 mL) was added under ice-cooling, and then, the mixture was stirred at the same temperature for 30 minutes. A sodium hydroxide aqueous solution (5 N, 24 mL) was added thereto, and the mixture was extracted with ethyl acetate, and then, the mixture was washed with brine, dried over magnesium sulfate, and thereafter the solvent was distilled off under reduced pressure to yield a crude product (1.1 g) of 3-[2,6-dimethoxy-4-(methoxymethyl)phenyl]-6-ethyl-2-methyl-7-nitrosopyrazolo[5,1-b][1,3 ]oxazole.
The obtained nitroso compound and 5% palladium-carbon (50% wet, 0.52 g) were suspended in ethyl acetate (35 mL), and the mixture was stirred for 1 hour under normal pressure of a hydrogen atmosphere. After replacement by nitrogen, the mixture was filtered by Celite, and washed with ethyl acetate. The solvent was distilled off under reduced pressure, and the residue was purified by silica-gel column chromatography (ethyl acetate/n-heptane: 75% to methanol/ethyl acetate: 2%) to yield the title compound (0.58 g, 1.7 mmol).
¹H-NMR (CDCl₃) δ: 1.24 (t, J=7.6 Hz, 3H), 2.19 (s, 3H), 2.50 (br.s, 2H), 2.68 (q, J=7.6 Hz, 2H), 3.44 (s, 3H), 3.79 (s, 6H), 4.48 (s, 2H), 6.62 (s, 2H).

### [Example 5] N-(cyclopropylmethyl)-3-[2,6-dimethoxy-4-(methoxymethyl)phenyl]-6-ethyl-2-methyl-N-(t etrahydro-2H-pyran-4-ylmethyl)pyrazolo[5,1-b][1,3]oxazole-7-amine

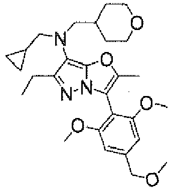

To a methanol solution (2 mL) of 3-[2,6-dimethoxy-4-(methoxymethyl)phenyl]-6-ethyl-2-methylpyrazolo[5,1-b][1,3]oxazole-7-amine (35 mg, 0.10 mmol) and tetrahydro-2H-pyran-4-carbaldehyde (13 mg, 0.11 mmol) were added acetic acid (60 µL, 1.1 mmol) and α-picoline borane complex (28 mg, 0.25 mmol) under ice-cooling, and the mixture was stirred for 30 minutes at room temperature, and then, cyclopropanecarboxyaldehyde (10 µL, 0.13 mmol) and α-picoline borane complex (16 mg, 0.15 mmol) were added thereto. After the mixture was stirred for 2.5 hours at room temperature, the solvent was distilled off under reduced pressure. A saturated sodium hydrogen carbonate aqueous solution was added thereto, the mixture was extracted with ethyl acetate, and then, the mixture was washed with brine, dried over magnesium sulfate, and the solvent was distilled off under reduced pressure. The residue was purified by silica-gel column chromatography (ethyl acetate/n-heptane: 20% to 50%) to yield the title compound (27 mg, 0.055 mmol).
¹H-NMR (CDCl₃) δ: 0.02-0.09 (m, 2H), 0.38-0.47 (m, 2H), 0.83-0.97 (m, 1H), 1.21 (t, J=7.6 Hz, 3H), 1.22-1.35 (m, 2H), 1.57-1.71 (m, 1H), 1.73-1.82 (m, 2H), 2.20 (s, 3H), 2.65 (q, J=7.6 Hz, 2H), 2.70-2.77 (m, 2H), 2.84-2.92 (m, 2H), 3.30-3.42 (m, 2H), 3.45 (s, 3H), 3.81 (s, 6H), 3.90-4.00 (m, 2H), 4.48 (s, 2H), 6.63 (s, 2H).

The compounds of Example 6 to Example 9 below were synthesized similarly to Example 5.

### [Example 6] 3-[2,6-dimethoxy-4-(methoxymethyl)phenyl]-6-ethyl-N-isobutyl-2-methyl-N-(tetrahydro-2 H-pyran-4-ylmethyl)pyrazolo[5,1-b][1,3]oxazole-7-amine

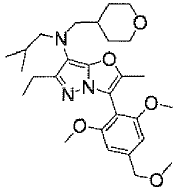

¹H-NMR (CDCl₃) δ: 0.88-0.96 (m, 6H), 1.20-1.33 (m, 2H), 1.21 (t, J=7.6 Hz, 3H), 1.59-1.72 (m, 2H), 1.73-1.82 (m, 2H), 2.20 (s, 3H), 2.63 (q, J=7.6 Hz, 2H), 2.63-2.68 (m, 2H), 2.72-2.77 (m, 2H), 3.31-3.40 (m, 2H), 3.45 (s, 3H), 3.82 (s, 6H), 3.91-3.99 (m, 2H), 4.48 (s, 2H), 6.63 (s, 2H).

### [Example 7] N,N-bis(cyclopropylmethyl)-3-[2,6-dimethoxy-4-(methoxymethyl)phenyl]-6-ethyl-2-methyl pyrazolo[5,1-b][1,3]oxazole-7-amine

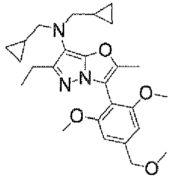

¹H-NMR (CDCl₃) δ: 0.02-0.11 (m, 4H), 0.35-0.45 (m, 4H), 0.84-0.98 (m, 2H), 1.24 (t, J=7.6 Hz, 3H), 2.19 (s, 3H), 2.71 (q, J=7.6 Hz, 2H), 2.79-2.88 (m, 4H), 3.44 (s, 3H), 3.80 (s, 6H), 4.48 (s, 2H), 6.62 (s, 2H).

### [Example 8] N-(cyclopropylmethyl)-3-[2,6-dimethoxy-4-(methoxymethyl)phenyl]-6-ethyl-2-methyl-N-(t etrahydrofuran-3-ylmethyl)pyrazolo[5,1-b][1,3]oxazole-7-amine

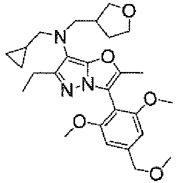

¹H-NMR (CDCl₃) δ: 0.03-0.12 (m, 2H), 0.39-0.48 (m, 2H), 0.85-0.98 (m, 1H), 1.22 (t, J=7.6 Hz, 3H), 1.60-1.73 (m, 1H), 1.90-2.02 (m, 1H), 2.20 (s, 3H), 2.27-2.41 (m, 1H), 2.64 (q, J=7.6 Hz, 2H), 2.72-2.79 (m, 2H), 2.90-2.99 (m, 1H), 3.01-3.10 (m, 1H), 3.45 (s, 3H), 3.57-3.65 (m, 1H), 3.67-3.89 (m, 3H), 3.805 (s, 3H), 3.810 (s, 3H), 4.48 (s, 2H), 6.63 (s, 2H).

### [Example 9] N-(cyclopropylmethyl)-3-[4-(ethoxymethyl)-2,6-dimethoxyphenyl]-6-ethyl-2-methyl-N-(tetr ahydrofuran-3-ylmethyl)pyrazolo[5,1-b][1,3]oxazole-7-amine

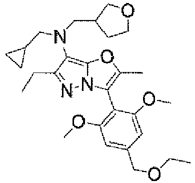

¹H-NMR (CDCl₃) δ: 0.03-0.11 (m, 2H), 0.40-0.49 (m, 2H), 0.86-0.98 (m, 1H), 1.22 (t, J=7.6 Hz, 3H), 1.29 (t, J=6.8 Hz, 3H), 1.60-1.72 (m, 1H), 1.91-2.01 (m, 1H), 2.20 (s, 3H), 2.29-2.41 (m, 1H), 2.64 (q, J=7.6 Hz, 2H), 2.71-2.80 (m, 2H), 2.90-2.99 (m, 1H), 3.01-3.10 (m, 1H), 3.57-3.65 (m, 1H), 3.59 (q, J=6.8 Hz, 2H), 3.67-3.76 (m, 1H), 3.78-3.88 (m, 2H), 4.52 (s, 2H), 6.64 (s, 2H).

### [Example 10] N-(cyclopropylmethyl)-3-[2,6-dimethoxy-4-(methoxymethyl)phenyl]-2,6-dimethyl-N-(tetrahydro-2H-pyran-4-ylmethyl)pyrazolo[5,1-b][1,3]oxazole-7-amine

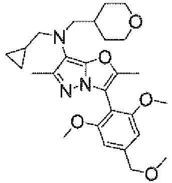

The titled compound was synthesized similarly to Examples 4 and 5 by using 3-methyl-2-pyrazolin-5-one.
¹H-NMR (CDCl₃) δ: 0.00-0.09 (m, 2H), 0.38-0.48 (m, 2H), 0.83-0.96 (m, 1H), 121-1.36 (m, 2H), 1.55-1.70 (m, 1H), 1.72-1.82 (m, 2H), 2.19 (s, 3H), 2.25 (s, 3H), 2.68-2.76 (m, 2H), 2.84-2.92 (m, 2H), 3.30-3.41 (m, 2H), 3.44 (s, 3H), 3.80 (s, 6H), 3.90-4.00 (m, 2H), 4.48 (s, 2H), 6.62 (s, 2H).

The compounds of Example 11 to Example 13 below were synthesized similarly to Example 10.

### [Example 11] 3-[2,6-dimethoxy-4-(methoxymethyl)phenyl]-N-isobutyl-2,6-dimethyl-N-(tetrahydro-2H-py ran-4-ylmethyl)pyrazolo[5,1-b][1,3]oxazole-7-amine

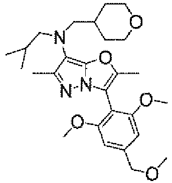

¹H-NMR (CDCl₃) δ: 0.87-0.97 (m, 6H), 1.19-1.35 (m, 2H), 1.56-1.70 (m, 2H), 1.73-1.82 (m, 2H), 2.19 (s, 3H), 2.24 (s, 3H), 2.62-2.69 (m, 2H), 2.72-2.79 (m, 2H), 3.31-3.41 (m, 2H), 3.44 (s, 3H), 3.81 (s, 6H), 3.90-4.01 (m, 2H), 4.48 (s, 2H), 6.62 (s, 2H).

### [Example 12] 3-[2,6-dimethoxy-4-(methoxymethyl)phenyl]-N-isobutyl-2,6-dimethyl-N-(tetrahydrofuran-3 -ylmethyl)pyrazolo[5,1-b][1,3]oxazole-7-amine

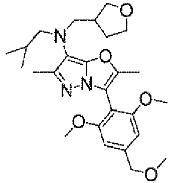

¹H-NMR (CDCl₃) δ: 0.88-0.97 (m, 6H), 1.57-1.71 (m, 2H), 1.91-2.03 (m, 1H), 2.20 (s, 3H), 2.24 (s, 3H), 2.27-2.40 (m, 1H), 2.63-2.72 (m, 2H), 2.77-2.82 (m, 1H), 2.89-2.98 (m, 1H), 3.44 (s, 3H), 3.57-3.64 (m, 1H), 3.68-3.76 (m, 1H), 3.77-3.88 (m, 2H), 3.801 (s, 3H), 3.805 (s, 3H), 4.48 (s, 2H), 6.62 (s, 2H).

### [Example 13] N-(cyclopropylmethyl)-3-[4-(ethoxymethyl)-2,6-dimethoxyphenyl]-2,6-dimethyl-N-(tetrahy dro-2H-pyran-4-ylmethyl)pyrazolo[5,1-b][1,3]oxazole-7-amine

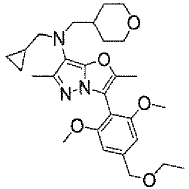

¹H-NMR (CDCl₃) δ: 0.00-0.10 (m, 2H), 0.38-0.47 (m, 2H), 0.83-0.96 (m, 1H), 1.20-1.37 (m, 2H), 1.28 (t, J=7.2 Hz, 3H), 1.53-1.70 (m, 1H), 1.72-1.82 (m, 2H), 2.19 (s, 3H), 2.25 (s, 3H), 2.69-2.77 (m, 2H), 2.84-2.92 (m, 2H), 3.30-3.40 (m, 2H), 3.59 (q, J=7.2 Hz, 2H), 3.80 (s, 6H), 3.90-4.00 (m, 2H), 4.52 (s, 2H), 6.63 (s, 2H).

### [Example 14] N-(cyclopropylmethyl)-3-[2,6-dimethoxy-4-(methoxymethyl)phenyl]-N-(1,3-dioxan-5-ylme thyl)-6-ethyl-2-methylpyrazolo[5,1-b][1,3]oxazole-7-amine

### (14a) tert-butyl (3-[2,6-dimethoxy-4-(methoxymethyl)phenyl]-6-ethyl-2-methylpyrazolo[5,1-b][1,3]oxazol-7-yl)carbamate

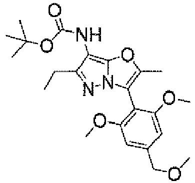

To a tetrahydrofuran solution (4 mL) of 3-[2,6-dimethoxy-4-(methoxymethyl)phenyl]-6-ethyl-2-methylpyrazolo[5,1-b][1,3]oxazole-7-amine (470 mg, 1.36 mmol) were added triethylamine (260 µL, 1.87 mmol) and di-*tert*-butyl dicarbonate (350 mg, 1.60 mmol), and the mixture was stirred at room temperature for 4 hours. A saturated sodium hydrogen carbonate aqueous solution was added to the reaction mixture, and the mixture was extracted with ethyl acetate, and then, the mixture was washed with brine, dried over magnesium sulfate, filtered, and thereafter, the solvent was distilled off under reduced pressure. The residue was purified by medium-pressure silica-gel column chromatography (ethyl acetate/n-heptane: 50% to 80%) to yield the title compound (550 mg, 1.23 mmol).
¹H-NMR (CDCl₃) δ: 1.21 (t, J=7.6 Hz, 3H), 1.52 (s, 9H), 2.20 (s, 3H), 2.63 (q, J=7.6 Hz, 2H), 3.44 (s, 3H), 3.78 (s, 6H), 4.48 (s, 2H), 5.65 (br.s, 1H), 6.61 (s, 2H).

### (14b) N-(cyclopropylmethyl)-3-[2,6-dimethoxy-4-(methoxymethyl)phenyl]-N-(1,3-dioxan-5-ylme thyl)-6-ethyl-2-methylpyrazolo[5,1-b][1,3]oxazole-7-amine

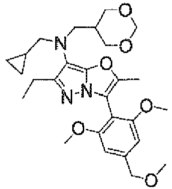

To a dimethyl sulfoxide solution (3 mL) of *tert*-butyl (3-[2,6-dimethoxy4-(methoxmethyl)phenyl]-6-ethyl-2-methylpyrazolo[5,1-b][1,3]oxazol-7-yl)carbamate (100 mg , 0.22 mmol) were added sodium hydroxide (powder) (36 mg, 0.90 mmol) and 5-iodomethyl-[1,3]dioxane (80 mg, 0.35 mmol) at room temperature, and the mixture was stirred for 2 hours at 50°C. A saturated ammonium chloride aqueous solution was added thereto at room temperature, the mixture was extracted with ethyl acetate, and then, the mixture was washed with brine, dried over magnesium sulfate, filtered, and thereafter the solvent was distilled off under reduced pressure to yield a crude product (135 mg) of *tert*-butyl (3-[2,6-dimethoxy-4-(methoxymethyl)phenyl]-*N*-(1,3-dioxan-5-ylmethyl)-6-ethyl-2-methylp yrazolo[5,1-b][1,3]oxazol-7-yl)carbamate.
The resulting crude product was dissolved in methylene chloride (3 mL), and trifluoroacetic acid (1 mL) was added thereto, and then, the mixture was stirred at room temperature for 1 hour, and the solvent was distilled off under reduced pressure.
To a methanol solution (3 mL) of the residue were added acetic acid (100 µL, 1.75 mmol), cyclopropanecarboxyaldehyde (20 µL, 0.27 mmol) and α-picoline borane complex (32 mg, 0.29 mmol) at room temperature. After the mixture was stirred at room temperature for 3 hours, the solvent was distilled off under reduced pressure. A saturated sodium hydrogen carbonate aqueous solution was added thereto, the mixture was extracted with ethyl acetate, and then, the mixture was washed with brine, dried over magnesium sulfate, and the solvent was distilled off under reduced pressure. The residue was purified by NH silica-gel column chromatography (ethyl acetate/n-heptane: 10% to 25%) to yield the title compound (91 mg, 0.18 mmol).
¹H-NMR (CDCl₃) δ: 0.03-0.11 (m, 2H), 0.40-0.49 (m, 2H), 0.85-0.97 (m, 1H), 1.21 (t, J=7.6 Hz, 3H), 1.91-2.02 (m, 1H), 2.19 (s, 3H), 2.63 (q, J=7.6 Hz, 2H), 2.70-2.79 (m, 2H), 2.98-3.07 (m, 2H), 3.44 (s, 3H), 3.61-3.71 (m, 2H), 3.81 (s, 6H), 4.02-4.12 (m, 2H), 4.48 (s, 2H), 4.75 (d, J=6.4 Hz, 1H), 4.91 (d, J=6.4 Hz, 1H), 6.63 (s, 2H).

The compounds of Example 15 and Example 16 below were synthesized similarly to Example 14.

### [Example 15] N-butyl-3-[2,6-dimethoxy-4-(methoxymethyl)phenyl]-6-ethyl-2-methyl-N-(tetrahydro-2H-p yran-4-yl)pyrazolo[5,1-b][1,3]oxazole-7-amine

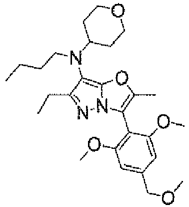

¹H-NMR (CDCl₃) δ:0.83-0.92 (m, 3H), 1.20 (t, J=7.6 Hz, 3H), 1.28-1.40 (m, 4H), 1.53-1.68 (m, 2H), 1.78-1.87 (m, 2H), 2.20 (s, 3H), 2.62 (q, J=7.6 Hz, 2H), 2.90-3.04 (m, 3H), 3.33-3.44 (m, 2H), 3.45 (s, 3H), 3.82 (s, 6H), 3.95-4.04 (m, 2H), 4.48 (s, 2H), 6.63 (s, 2H).

### [Example 16] N-(cyclopropylmethyl)-3-[2,6-dimethoxy-4-(methoxymethyl)phenyl]-6-ethyl-2-methyl-N-(t etrahydro-2H-pyran-4-yl)pyrazolo[5,1-b][1,3]oxazole-7-amine

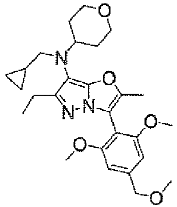

¹H-NMR (CDCl₃) δ: -0.04-0.05 (m, 2H), 0.32-0.41 (m, 2H), 0.78-0.90 (m, 1H), 1.22 (t, J=7.6 Hz, 3H), 1.51-1.68 (m, 2H), 1.78-1.89 (m, 2H), 2.19 (s, 3H), 2.66 (q, J=7.6 Hz, 2H), 2.78-2.87 (m, 2H), 3.03-3.16 (m, 1H), 3.33-3.46 (m, 2H), 3.44 (s, 3H), 3.81 (s, 6H), 3.95-4.05 (m, 2H), 4.48 (s, 2H), 6.63 (s, 2H).

### [Pharmacological Test Example]

The binding capacities of the compounds of the present invention for CRF1 receptor (CRFR1) were evaluated. The test methods and results were as described below.

### Test Example 1

### <CRFR1 Binding Test>

### (1) Preparation of CRFR1-Expressing Cells

The membrane fraction of human CRFR1 high-expressing cells was used as the material for a CRFR1 binding experiment. The CRFR1-expressing cells were prepared in the following manner. The full-length CRFR1 gene was obtained by PCR by using human brain cDNA library (QuickClone (TM), Clontech). The obtained DNA fragment was inserted into a cloning vector and the base sequence was confirmed. cDNA having the proper base sequence was linked to an expression vector (pcDNA3.1 (TM), Invitrogen). The CRFR1 expression vector was genetically introduced into HEK293 cell, and the resistant cells which proliferated in culture medium containing G418 (1 mg/ml) were cloned by the limiting dilution method. Out of the cloned cells, cells having high binding capacity between the membrane fraction per unit protein and sauvagine were selected according to the following binding experiment, and the selected cells were used for the experiments.

### (2) Preparation of Membrane Fraction

The cloned cells obtained in (1) were collected and suspended in ice-cooled membrane buffer (50 mM Tris-HCl, 5 mM MgCl₂, 2 mM EGTA, 1 mM DTT, protease inhibitor cocktail (COMPLETE™, Roche Diagnostics) pH 7.3), and then the cells were disrupted with a Polytron (KINEMATICA) while cooling on ice (level 5, 10 seconds, 2-5 times, ice-cooling) and then centrifuged (2,000 rpm, 5 minutes, 4°C), followed by collecting the supernatant. Membrane buffer was added to the precipitate, and the mixture was subjected to Polytron treatment (same conditions as mentioned above) and centrifuged (conditions as mentioned above), and the obtained supernatant was collected and combined with the previous supernatant. This was centrifuged (13,000 rpm (18,000 xg), 30 minutes, 4°C) to prepare cell membranes. The precipitated cell membranes were suspended in membrane buffer and disrupted with a Polytron (level 5,10 seconds, 3-5 times, ice-cooling) to prepare a dispersed suspension. The protein assay was carried out.
The following method (1) or (2) was carried out for use as the cell membrane fraction.
(1) The above dispersed suspension was diluted with membrane buffer containing 0.1% BSA to a protein concentration of 200 µg/ml, for use as the cell membrane fraction.
(2) The above dispersed suspension was freeze-preserved, and if necessary, thawed, re-dispersed and diluted for use as the cell membrane fraction.

### (3) Binding Experiment:

A binding competition experiment with CRF was conducted by the SPA (GE Healthcare) method using a 96-well plate. Five µg of the cell membrane fraction protein, 1 mg of SPA beads and 100 pM ¹²⁵I-CRF (Perkin Elmer) were allowed to stand at room temperature for at least two hours in the presence of a test compound, and the radioactivity of each well after centrifugation (1,200 rpm (260 × g), five minutes, room temperature) was measured with a TopCount (registered trademark; Perkin Elmer).

### (4) Calculation of Binding Capacity

The radioactivity with addition of a 4,000-fold excess of non-radioactive sauvagine as the nonspecific binding was subtracted from each value, and the resulting value was expressed as a percentage (% of control), with 100% as the radioactivity without addition of the test compound (control). The IC₅₀ value was calculated from a binding inhibition curve in which the abscissa axis shows the test compound concentration and the ordinate axis shows % (% of control).

### <Test Results>

As shown in the following table, the compounds of the present invention exhibit excellent binding capacity with respect to CRFR1.

**[Table 1]**

| Compound No. (Example No.) | CRF1 receptor binding capacity IC₅₀(nM) |
|---|---|
| 3 | 100 |
| 5 | 32 |
| 6 | 43 |
| 7 | 21 |
| 8 | 77 |
| 9 | 44 |
| 10 | 71 |
| 11 | 50 |
| 12 | 79 |
| 13 | 61 |
| 14 | 121 |
| 15 | 98 |
| 16 | 102 |

### Test Example 2

### <Evaluation of Anxiolytic Effect in Light/Dark Box Test in Mice>

### (1) Test Procedure:

The light/dark box test in mice was carried out according to a modified method of Belzung C., Misslin R., and Vogel E. et al. (Reference; Behavioural effects of the benzodiazepine receptor partial agonist RO16-6028 in mice, Psychopharmacology, 97, 388-391, 1989). The test apparatus used in this test was a light/dark box including a covered black acrylic box (dark box; 15 × 10 × 20 cm), a white acrylic box with top opened (light box; 15 × 20 × 20 cm) and a black acrylic tunnel (10 × 7 × 4.5 cm) that connects the dark box and the light box and enables a mouse to freely move back and forth between the dark box and light box. In this test apparatus, however, a transparent acrylic plate was used for the front side (20 × 20 cm) and back side (20 × 20 cm) of the light box to allow observation of the behavior. After setting illumination so that the light intensity of the floor surface of the light box became 150 Lux, 5-week-old male Balb/c mice (purchased from Nihon Charles River) were introduced into the dark box and the test was started. In the test, the tested compound was suspended in 5% dimethyl sulfoxide, 5% Cremopor EL and 90% physiological saline and orally administered to the test animals one hour prior to the start of the test.

### (2) Calculation of Anxiolytic Effect:

The behavior of the mice was observed for 5 minutes after the start of the test. The time spent in the light box was measured as an index of the anxiolytic effect, with "spend in the light box" defined as the state in which all limbs of the mice were on the floor of the light box. The minimum dose which significantly prolonged the time spent in the light box in comparison with that of vehicle-treated group was determined as the minimum effective dose (MED). The statistical significance between the vehicle-treated group and the test compound-treated groups was analyzed by one-way layout analysis of variance followed by Dunnett multiple comparison when multiple doses were set for the same test, and by the Mann-Whitney U test when only one dose was set.

### <Test Results>

The compounds of Examples 5 and 10 exhibited an excellent anxiolytic effects in the light/dark box test in mice, with statistically significant effects when the dose was 30 mg/kg (oral administration).

**[Table 2]**

| Compound No. (Example No.) | Effective Dose (mg/kg) |
|---|---|
| 5 | 30 |
| 10 | 30 |

### Industrial Applicability

The present invention can provide pharmaceutical compositions comprising 3-phenylpyrazolo[5,1-b]thiazole compounds or pharmacologically acceptable salts thereof, which exhibit CRF receptor antagonism. The compounds or pharmacologically acceptable thereof according to the present invention have excellent CRF receptor antagonism, and sufficient pharmacological activity, safety and pharmacokinetic properties as medicines.

The pharmaceutical compositions of the present invention are useful for treatment or prevention of diseases associated with CRF and/or CRF receptors, and are particularly useful as therapeutic or prophylactic agents for depression, depressive symptoms, anxiety, irritable bowel syndrome, sleep disorder, insomnia, alcohol dependence, alcohol withdrawal symptoms, drug dependence, drug withdrawal symptoms, stress-related gastrointestinal dysfunction, anorexia nervosa, eating disorder, postoperative ileus, ischemic neuropathy, apoplexy, excitotoxic neuropathy, convulsion, epilepsy, hypertension, schizophrenia, bipolar disorder, dementia, or the like.

## Claims

1. A compound represented by the formula (I) or pharmacologically acceptable salt thereof: 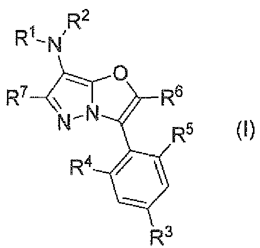 wherein R¹ and R² are the same or different and are (a) a hydrogen atom, (b) a C1-6 alkyl group, (c) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, (d) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (e) R¹ and R², together with a nitrogen atom to which they are attached, form a pyrrolidinyl group, a piperidinyl group or a morpholinyl group;
R³, R⁴ and R⁵ are the same or different and are a hydrogen atom, a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
R⁶ is a hydrogen atom or a C 1-6 alkyl group; and
R⁷ is a C1-6 alkyl group, a C1-6 alkoxy group or a C1-6 alkylthia group.

2. The compound or pharmacologically acceptable salt thereof according to claim 1,
wherein R¹ is a hydrogen atom, a C1-6 alkyl group, a C3-6 cycloalkyl group or a C3-6 cycloalkyl-C1-6 alkyl group;
R² is (a) a hydrogen atom, (b) a C1-6 alkyl group, (c) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (d) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group;
R³ is a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
R⁴ is a hydrogen atom, a C1-6 alkyl group or a C1-6 alkoxy group; and
R⁵ is a halogen atom, a C 1-6 alkyl group or a C 1-6 alkoxy group.

3. The compound or pharmacologically acceptable salt thereof according to claim 2,
wherein R¹ is a hydrogen atom, a C1-6 alkyl group, a cyclopropyl group or a cyclopropylmethyl group; and
R² is a hydrogen atom, a C1-6 alkyl group, a cyclopropylmethyl group, a tetrahydropyranyl group, a tetrahydropyranylmethyl group, a dioxanylmethyl group or a tetrahydrofurylmethyl group.

4. The compound or pharmacologically acceptable salt thereof according to claim 2,
wherein R³ is a halogen atom, methoxy or a C1-6 alkoxymethyl group;
R⁴ is a hydrogen atom or a methoxy group; and
R⁵ is a halogen atom or a methoxy group.

5. The compound or pharmacologically acceptable salt thereof according to claim 2,
wherein R⁶ is a hydrogen atom or a methyl group; and
R⁷ is a methyl group or an ethyl group.

6. The compound or pharmacologically acceptable salt thereof according to claim 1,
wherein R¹ is a hydrogen atom, a C1-6 alkyl group, a cyclopropyl group or a cyclopropylmethyl group;
R² is a hydrogen atom, a C1-6 alkyl group, a cyclopropylmethyl group, a tetrahydropyranyl group, a tetrahydropyranylmethyl group, a dioxanylmethyl group or a tetrahydrofurylmethyl group;
R³ is a halogen atom, a methoxy group or a C1-6 alkoxymethyl group;
R⁴ is a hydrogen atom or a methoxy group;
R⁵ is a halogen atom or a methoxy group;
R⁶ is a hydrogen atom or a methyl group; and
R⁷ is a methyl group or an ethyl group.

7. A pharmaceutical composition comprising a compound or pharmacologically acceptable salt thereof according to claim I as an active ingredient.

8. The pharmaceutical composition according to claim 7, which is a CRF1 receptor antagonist.

9. A therapeutic or a prophylactic agent for depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction, anorexia nervosa, eating disorders, postoperative ileus, ischemic neuropathy, apoplexy, excitotoxic neuropathy, convulsion, epilepsy, hypertension, schizophrenia, bipolar disorder or dementia, comprising a compound or pharmacologically acceptable salt thereof according to claim 1 as an active ingredient.

10. A therapeutic or prophylactic agent for depression, depressive symptoms, anxiety, irritable bowel syndromes, sleep disorders, insomnia, alcohol dependence, alcohol withdrawal symptoms, drug dependence, drug withdrawal symptoms, stress-related gastrointestinal dysfunction or dementia, comprising a compound or pharmacologically acceptable salt thereof according to claim 1 as an active ingredient.

11. A therapeutic or a prophylactic agent for depression, depressive symptoms, anxiety or irritable bowel syndromes, comprising a compound or pharmacologically acceptable salt thereof according to claim 1 as an active ingredient.

12. A compound represented by the formula (Ia) or pharmacologically acceptable salt thereof: 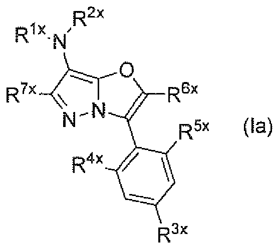 wherein R^{1x} and R^{2x} are the same or different and are (a) a C1-6 alkyl group, (b) a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group, (c) a C1-6 alkyl group substituted with a cyclic group selected from a C3-6 cycloalkyl group, a tetrahydropyranyl group, a dihydropyranyl group, a tetrahydrofuryl group, a dioxanyl group, a tetrahydrothienyl group, a dithianyl group and a hexahydrothiepinyl group or (d) R^{1x} and R^{2x}, together with a nitrogen atom to which they are attached, form a pyrrolidinyl group, a piperidinyl group or a morpholinyl group;
R^{3x}, R^{4x} and R^{5x} are the same or different and are a hydrogen atom, a C1-6 alkyl group, C3-6 cycloalkyl, a C1-6 alkoxy group, a C1-6 alkoxy-C1-6 alkyl group, a C3-6 cycloalkoxy-C1-6 alkyl group or a halogen atom;
R^{6x} is a hydrogen atom or a C1-6 alkyl group; and
R^{7x} is a C1-6 alkyl group, a C 1-6 alkoxy group or a C 1-6 alkylthio group,
with the proviso that when R^{7x} is a C1-6 alkyl group, at least one of R^{1x} and R^{2x} is (a) a cyclic group selected from a tetrahydropyranyl group, a dihydropyranyl group, a tetrahydrofuryl group and a dioxanyl group or (b) a C1-6 alkyl group substituted with a cyclic group selected from a tetrahydropyranyl group, a dihydropyranyl group, a tetrahydrofuryl group and a dioxanyl group.

13. The compound or pharmacologically acceptable salt thereof according to claim 12,
wherein R^{1x} is a C1-6 alkyl group, a C3-6 cycloalkyl group or a C3-6 cycloalkyl-C1-6 alkyl group;
R^{2x} is (a) a cyclic group selected from a tetrahydropyranyl group, a dihydropyranyl group, a tetrahydrofuryl group and a dioxanyl group or (b) a C1-6 alkyl group substituted with a cyclic group selected from a tetrahydropyranyl group, a dihydropyranyl group, a tetrahydrofuryl group and a dioxanyl group;
R^{3x} is a C1-6 alkyl group, a C3-6 cycloalkyl group, a C1-6 alkoxy group, a C1-6 alkoxy-C1-6 alkyl group, a C3-6 cycloalkoxy-C1-6 alkyl group or a halogen atom;
R^{4x} is a hydrogen atom, a C1-6 alkyl group or a C1-6 alkoxy group; and
R^{5x} is a halogen atom, a C1-6 alkyl group or a C1-6 alkoxy group.

14. The compound or pharmacologically acceptable salt thereof according to claim 13,
wherein R^{1x} is a C1-6 alkyl group, a cyclopropyl group or a cyclopropylmethyl group; and
R^{2x} is a tetrahydropyranyl group, a tetrahydropyranylmethyl group, a dioxanylmethyl group or a tetrahydrofurylmethyl group.

15. The compound or pharmacologically acceptable salt thereof according to claim 13,
wherein R^{3x} is a halogen atom, a methoxy group or a C 1-6 alkoxymethyl group;
R^{4x} is a hydrogen atom or a methoxy group; and
R^{5x} is a halogen atom or a methoxy group.

16. The compound or pharmacologically acceptable salt thereof according to claim 15, wherein R^{3x} is a C1-6 alkoxymethyl group.

17. The compound or pharmacologically acceptable salt thereof according to claim 13,
wherein R^{6x} is a hydrogen atom or a methyl group; and
R^{7x} is a methyl group or an ethyl group.

18. The compound or pharmacologically acceptable salt thereof according to claim 13,
wherein R^{1x} is a C1-6 alkyl group, a cyclopropyl group or a cyclopropylmethyl group;
R^{2x} is a tetrahydropyranyl group, a tetrahydropyranylmethyl group, a dioxanylmethyl group or a tetrahydrofurylmethyl group;
R^{3x} is a C1-6 alkoxymethyl group,
R^{4x} is a hydrogen atom or a methoxy group;
R^{5x} is a halogen atom or a methoxy group;
R^{6x} is a hydrogen atom or a methyl group; and
R^{7x} is a methyl group or an ethyl group.
